# EUROPEAN PATENT APPLICATION

(11) **EP 2 062 651 A2**
(43) Date of publication of application: **27.05.2009**
(21) Application number: 09003225.1
(22) Date of filing: 28.12.2005
(51) Int. Cl.: B05B 5/08, A61M 11/00

(54) **Electrostatic spraying device**

(30) Priority: 28.12.2004 JP 2004380593; 28.12.2004 JP 2004380366
(62) Divisional of application: 05844866.3
(71) Applicant: Daikin Industries, Ltd., Osaka 530-8323 (JP)
(72) Inventor: Okumoto, Mamoru, Sakai-shi Osaka 5918511 (JP); Koyama, Noboru, Sakai-shi Osaka 5918511 (JP)
(74) Representative: Goddar, Heinz J.

(57) **Abstract**

Disclosed is an electrostatic spraying device **(10)** which has a spray cartridge **(15),** a power supply **(16),** and a controller **(17).** In the spray cartridge **(15),** a nozzle unit **(30)** is mounted to the lower part of a solution tank **(20).** The tip of a spray nozzle **(31)** of the nozzle unit **(30)** is situated below a liquid level **(51)** in the solution tank **(20).** There is a head difference between the tip of the spray nozzle **(31)** and the liquid level **(51)** in the solution tank **(20),** which head difference causes water solution in the solution tank **(20)** to be supplied into the spray nozzle **(31).** Upon application of a voltage to the spray nozzle **(31)** from the power supply **(16),** the water solution is sprayed from the tip of the spray nozzle **(31).**

## Description

### TECHNICAL FIELD

This invention relates to an electrostatic spraying device for atomizing and spraying liquid by electro hydrodynamic.

### BACKGROUND ART

For many years, electrostatic spraying devices configured to atomize and spray liquid by EHD (Electro Hydrodynamic) have been well known in the art. In a typical electrostatic spraying device, an electric field is formed in the vicinity of the opening end of a thin-diameter tube and liquid in the thin-diameter pipe is atomized and sprayed through the use of the non-uniform property of the electric field.

Inhaling devices formed by electrostatic spraying devices are disclosed in JP-A-1987-197071 and JP-T-2002-532163. An inhaling device of this type is adapted and configured to atomize a therapeutic product, used in treating asthma, bronchitis et cetera, into minute liquid droplets for inhalation through the user's nose. In addition, JP-T-2003-507166 discloses a spraying device of the hand-held type which is composed of an electrostatic spraying device. In this spraying device, a cosmetic product, e.g., a foundation in liquid form, perfume water et cetera, is atomized into minute liquid droplets for application onto the user's skin.

In these devices (inhaling device and spraying device) disclosed in the patent documents, an electric filed is formed in the vicinity of the tip of a nozzle and liquid present in the nozzle tip is atomized and discharged. At the same time, in these devices (inhaling device and spraying device), an extruding mechanism using a pump, an electric motor et cetera is employed to supply liquid to be atomized to the nozzle tip at which the liquid supplied is atomized.

### DISCLOSURE OF THE INVENTION

### PROBLEMS WHICH THE INVENTION SEEKS TO OVERCOME

As described above, electrostatic spraying devices of the conventional type as set forth in the patent documents employ a pump, an electric motor, or some like means to supply liquid to the tip of a thin-diameter tube at which the liquid supplied is atomized. That is, these devices require a component member (pump, electric motor, or some like means) for liquid supply. Consequently, the electrostatic spraying device may become complicated in configuration and expensive to manufacture. In addition, if the electric motor (pump) breaks down, it becomes impossible to spray liquid, thereby causing a problem that it becomes difficult to ensure the reliability of the electrostatic spraying device.

With these problems in mind, the present invention was made. Accordingly, an object of the present invention is to provide an electrostatic spraying device which has a simplified configuration, which is reduced in production cost, and which is improved in reliability.

### MEANS FOR OVERCOMING THE PROBLEMS

The present invention provides, as a first aspect, an electrostatic spraying device which comprises: a reservoir member **(20)** to store liquid; a tubular nozzle member **(31)** which is in fluid communication with an internal space of the reservoir member **(20)**; a first electrode **(31, 37)** which is in contact with liquid present in the nozzle member **(31);** and a second electrode **(35, 38, 60, 61)** which is insulated from the liquid in the nozzle member **(31),** wherein upon creation of an electric potential difference between the first electrode **(31, 37)** and the second electrode **(35, 38, 60, 61)** liquid is sprayed from the tip of the nozzle member **(31)**. And the tip of the nozzle member **(31)** is disposed at a lower position than a liquid level **(51)** in the reservoir member **(20).**

In the first aspect, fluid within the reservoir member **(20)** flows into the nozzle member **(31)**. Upon creation of an electric potential difference between the first electrode **(31, 37)** and the second electrode **(35, 38, 60, 61),** the liquid supplied is atomized in the tip of the nozzle member **(31),** and the liquid now in the form of minute liquid droplets is sprayed from the tip of the nozzle member **(31)**. In the electrostatic spraying device of the first aspect, the tip of the nozzle member **(31)** is disposed such that its position is lower than the liquid level **(51)** in the internal space of the reservoir member **(20),** and there is a head difference between the tip of the nozzle member **(31)** and the liquid level **(51)** in the reservoir member **(20)**. Owing to this head difference, liquid within the reservoir member **(20)** is supplied to the tip of the nozzle member **(31)** at which the liquid is atomized.

The present invention provides, as a second aspect
an electrostatic spraying device in which said electrostatic spraying device is configured such that, in a state where the first electrode **(31, 37)** and the second electrode **(35, 38, 60, 61)** become the same electric potential as each other to thereby provide a balance between liquid pressure and surface tension at a gas-liquid interface **(52)** of the tip of the nozzle member **(31)**, liquid outflow from the tip of the nozzle member **(31)** is prevented from occurring.

In the second aspect, when entering such a state that there is no electric potential difference between the first electrode **(31, 37)** and the second electrode **(35, 38, 60, 61)**, this state establishes, in the gas-liquid interface **(52)** formed in the tip of the nozzle member **(31),** a balance between liquid pressure and surface tension both acting on the gas-liquid interface **(52)**. In other words, in order to establish a balance between liquid pressure and surface tension when the first electrode **(31, 37)** and the second electrode **(35, 38, 60, 61)** are at the same electric potential, maximum values for the height of the liquid level **(51)** in the reservoir member **(20),** inside diameters for the nozzle member **(31)** et cetera are set depending on the physicality of liquid to be atomized such as viscosity. And in a state where there is provided a balance between liquid pressure and surface tension at the gas-liquid interface **(52)** of the tip of the nozzle member **(31)**, no liquid will flow out of the tip of the nozzle member **(31),** even when liquid pressure acts on the gas-liquid interface **(52)** of the tip of the nozzle member **(31).**

The present invention provides, as a third aspect an electrostatic spraying device in which the tip of the nozzle member **(31)** is disposed at a lower position than a bottom surface **(22)** of the reservoir member **(20)**.

In the third aspect, the tip of the nozzle member **(31)** is arranged to be below the bottom surface **(22)** of the reservoir member **(20)**. Therefore, even when almost all of the liquid flows out of the reservoir member **(20)**, the tip of the nozzle member **(31)** is kept filled with liquid.

The present invention provides, as a fourth aspect according to the first aspect, an electrostatic spraying device which further comprises: a power supply **(16)** which applies a voltage to at least the first electrode **(31, 37)** to create an electric potential difference between the first electrode **(31, 37)** and the second electrode **(35, 38, 60**, **61)**; and a controller means **(17)** by which the power supply **(16)** is repeatedly turned on and off and which controls, in response to variation in the height of the liquid level **(51)** in the reservoir member **(20)**, the duty ratio of the length of time for which the power supply **(16)** is turned on and the length of time for which the power supply **(16)** is turned off.

In the fourth aspect, the controller means **(17)** repeatedly performs ON/OFF of the power supply **(16).** When the power supply **(16)** is turned on, a voltage is applied to at least the first electrode **(31, 37).** Owing to this voltage application, there is created an electric potential difference between the first electrode **(31, 37)** and the second electrode **(35, 38, 60, 61),** and spray from the tip of the nozzle member **(31)** starts. On the other hand, when the power supply **(16)** is turned off, the first electrode **(31, 37)** and the second electrode **(35, 38, 60, 61)** are placed at the same electric potential, and spray from the nozzle member **(31)** stops. If the duty ratio of the ON and OFF times of the power supply **(16)** is changed, the length of time per unit time, for which spray from the nozzle member **(31)** is performed, is varied, and the amount of spray from the nozzle member (**31)** is varied. In the electrostatic spraying device of the fourth aspect, the controller means **(17)** controls, in response to variation in the height of the liquid level **(52)** in the reservoir member **(20)**, the duty ratio of the ON and OFF times of the power supply **(16)**.

The present invention provides, as a fifth aspect according to the first aspect, an electrostatic spraying device in which the second electrode **(35)** is formed in a circular ring shape, the second electrode **(35)** being arranged coaxially with respect to the nozzle member **(31)**.

In the fifth aspect, the second electrode **(35)** is shaped like a circular ring. The second electrode **(35)** is arranged to be oriented such that its central axis corresponds to the central axis of the nozzle member **(31).**

The present invention provides, as a sixth aspect according to the first aspect, an electrostatic spraying device in which the second electrode **(60, 61)** is provided with a head end **(65)**, the head end **(65)** being arranged to be positioned lateral to the nozzle member **(31).**

In the sixth aspect, the second electrode **(60, 61)** is provided with the head end **(65).** The head end **(65)** is situated lateral to the nozzle member **(31).** When there is created an electric potential difference between the first electrode **(31, 37)** and the second electrode **(60, 61)**, an electric field is formed between the head end **(65)** of the second electrode **(60, 61)** and the first electrode **(31, 37),** and liquid is sprayed from the nozzle member **(31).**

The present invention provides, as a seventh aspect according to the sixth aspect, an electrostatic spraying device in which the second electrode **(60)** is formed in a rod shape, the second electrode **(60)** having a tip which becomes the head end **(65)**.

In the seventh aspect, the tip of the second electrode **(60)** formed in a rod shape becomes the head end **(65).** In other words, when there is created an electric potential difference between the first electrode **(31, 37)** and the second electrode **(60, 61),** an electric field is formed between the tip of the second electrode **(60, 61)** and the first electrode **(31, 37).**

The present invention provides, as an eighth aspect according to the first aspect, an electrostatic spraying device in which: the electrostatic spraying device further comprises a power supply **(16)** which applies a voltage to at least the first electrode **(31, 37)** to create an electric potential difference between the first electrode **(31, 37)** and the second electrode **(35, 38, 60, 61)**; and in a state where the power supply **(16)** is turned on the electric potential difference between the first electrode **(31, 37)** and the second electrode **(35, 38)** is not less than 3 kV but falls below a value at which a corona discharge starts occurring.

In the eighth aspect, upon the power supply **(16)** being turned on, a voltage is applied to at least the first electrode **(31, 37)**. This voltage application creates an electric potential difference between the first electrode **(31, 37)** and the second electrode **(35, 38, 60, 61),** and spray from the tip of the nozzle member **(31)** starts. On the other hand, upon the power supply **(16)** being turned off, the first electrode **(31, 37)** and the second electrode **(35, 38, 60, 61)** become the same electric potential as each other, and spray from the nozzle member **(31)** stops. In the state where the power supply **(16)** is turned on, the value of the electric potential difference created between the first electrode **(31, 37)** and the second electrode **(35, 38, 60, 61)** is set so as to fall within a predetermined range. In addition, the value of the electric potential difference, at which a corona discharge starts occurring between the first electrode **(31, 37)** and the second electrode **(35, 38, 60, 61),** varies depending on the distance between the first electrode **(31, 37)** and the second electrode **(35, 38, 60, 61).**

The present invention provides, as a ninth aspect according to any one of the first to eighth aspects, an electrostatic spraying device in which the nozzle member **(31)** is composed of an electrically conductive material, the nozzle member **(31)** serving also as the first electrode **(31, 37).**

In the ninth aspect, the nozzle member **(31)** composed of electrically conductive material serves also as the first electrode **(31, 37)**. In the electrostatic spraying device **(10)** of the ninth aspect, upon creation of an electric potential difference between the nozzle member **(31)** serving also as the first electrode **(31, 37)** and the second electrode **(35, 38, 60, 61),** liquid is atomized in the tip of the nozzle member **(31)** and then discharged.

The present invention provides, as a tenth aspect according to the ninth aspect, an electrostatic spraying device in which the distance between the tip of the nozzle member **(31)** and the second electrode **(35, 38, 60, 61)** is not less than 5 mm nor more than 20 mm.

In the tenth aspect, the distance between the tip of the nozzle member **(31)** which serves also as the first electrode **(31, 37)** and the second electrode **(35, 38, 60, 61)** is set to a value that falls within a predetermined range. If the distance between the tip of the nozzle member **(31)** and the second electrode **(35, 38, 60, 61)** is set too short, the value of the electric potential difference, at which a corona discharge starts occurring between the nozzle member **(31)** and the second electrode **(35, 38, 60, 61),** decreases. If trying to avoid occurrence of a corona discharge, it becomes difficult to create a sufficient electric potential difference between the nozzle member **(31)** and the second electrode **(35, 38, 60, 61)**, thereby giving rise to the possibility that the nozzle member **(31)** becomes unable to provide stable spray from its tip. On the other hand, if the distance between the tip of the nozzle member **(31)** and the second electrode **(35, 38, 60, 61)** is set too long, this excessively increases the value of the electric potential difference required to be created between the nozzle member **(31)** and the second electrode **(35, 38, 60, 61)**, thereby giving rise to the possibility of producing the problem that the power supply **(16)** for creating an electric potential difference grows in size. To overcome this problem, in consideration of the above, the distance between the tip of the nozzle member **(31)** and the second electrode **(35, 38, 60, 61)** is set to be not less than 5 mm nor more than 20 mm in the present aspect.

The present invention provides, as an eleventh aspect according to the ninth aspect, an electrostatic spraying device in which the tip of the nozzle member **(31)** has a thickness of not more than 0.2 mm.

In the eleventh aspect, the thickness of the tip of the nozzle member **(31)** is set to a certain value that falls within a predetermined range. In other words, the nozzle member **(31)** does not necessarily have a thickness of not more than 0.2 mm over its entire length. It suffices if the thickness of the endmost portion of the nozzle member **(31)** is not more than 0.2 mm. If the thickness of the tip of the nozzle member **(31)** is set excessively thick, this weakens the non-uniform property of the electric field to the tip of the nozzle member **(31),** therefore giving rise to the possibility that spray from the tip of the nozzle member **(31)** tends to become unstable. To cope with this, in consideration of the above, the thickness of the tip of the nozzle member **(31)** is set to be not more than 0.2 mm.

The present invention provides, as a twelfth aspect according to the ninth aspect, an electrostatic spraying device in which the nozzle member **(31)** has an inside diameter which is not less than 0.3 mm nor more than 1.0 mm.

In the twelfth aspect, the inside diameter of the nozzle member **(31)** is set so as to fall within a predetermined range. If the inside diameter of the nozzle member **(31)** is set excessively small, this excessively increases the surface tension of the gas-liquid interface **(52)** of the tip of the nozzle member **(31)**, thereby giving rise to the possibility that liquid is not stably sprayed from the tip of the nozzle member **(31).** On the other hand, if the inside diameter of the nozzle member **(31)** is set excessively small, this increases danger of causing the nozzle member **(31)** to clog. On the other hand, if the inside diameter of the nozzle member **(31)** is set excessively large, this excessively decreases the surface tension of the gas-liquid interface **(52)** of the tip of the nozzle member **(31),** therefore giving rise to the possibility that liquid atomization in the tip of the nozzle member **(31)** becomes unstable, also in this case. To cope with this, in consideration of the above, the inside diameter of the nozzle member **(31)** is not less than 0.3 mm nor more than 1.0 mm in the present aspect.

The present invention provides, as a thirteenth aspect according to the ninth aspect, an electrostatic spraying device in which the second electrode **(35, 38, 60, 61)** is disposed at a rearward position backed away from the tip towards the base end of the nozzle member **(31).**

In the thirteenth aspect, the second electrode **(35, 38, 60, 61)** is disposed not ahead of but behind the tip of the nozzle member **(31)**. If the second electrode **(35, 38, 60,** 61) is arranged ahead of the tip of the nozzle member **(31)**, this gives rise to the possibility that liquid droplets sprayed from the tip of the nozzle member **(31)** adhere to the second electrode **(35, 38, 60, 61).** To cope with this, in the present aspect, the second electrode **(35, 38, 60, 61)** is arranged at the back of the tip of the nozzle member **(31),** i.e., near the base end of the nozzle member **(31),** whereby liquid sprayed from the tip of the nozzle member **(31)** is prevented from adhering to the second electrode **(35, 38, 60, 61).**

The present invention provides, as a fourteenth aspect according to the thirteenth aspect, an electrostatic spraying device in which the distance for which the second electrode **(35, 38, 60, 61)** is backed away from the tip of the nozzle member **(31)** is not less than 3 mm nor more than 10 mm.

In the fourteenth aspect, the second electrode **(35, 38, 60, 61)** is disposed at a rearward position backed away from the tip of the nozzle member **(31)** by a predetermined distance. If the backed-away distance of the second electrode **(35, 38, 60, 61)** from the tip of the nozzle member **(31)** is set excessively short, this gives rise to the possibility of failing to form an electric field suitable for spray in the tip of the nozzle member **(31).** On the other hand, if the backed-away distance of the second electrode **(35, 38, 60, 61)** from the tip of the nozzle member **(31)** is set excessively long, this excessively increases the electric potential difference required to be created between the nozzle member **(31)** and the second electrode **(35, 38, 60, 61),** therefore giving rise to the possibility that the power supply **(16)** for creating an electric potential difference grows in size. To cope with this, in consideration of the above, the backed-away distance of the second electrode **(35, 38, 60, 61)** from the tip of the nozzle member **(31)** is not less than 3 mm nor more than 10 mm.

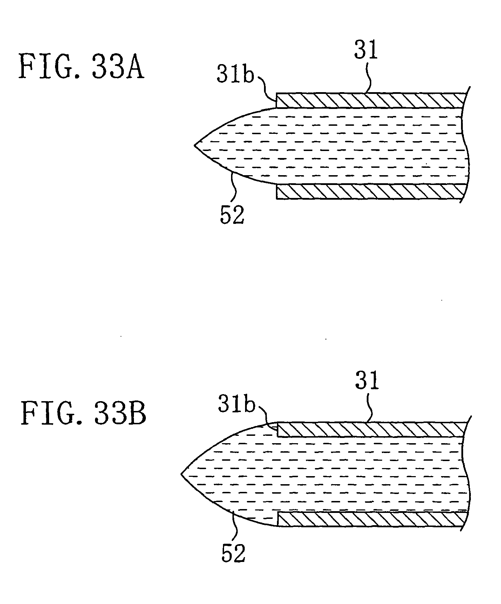

In the case where liquid is atomized in the electrostatic spraying device **(10)** of the present aspect, the gas-liquid interface **(52)** is extended by an electric filed into a cone shape in the tip of the nozzle member **(31).** The cone shaped gas-liquid interface **(52)** formed in the tip of the nozzle member **(31)** varies in size depending on the degree of wetness of the nozzle tip end surface. For example, in a state where the tip end surface of the nozzle member **(31)** is not wet at all, the gas-liquid interface **(52) i**s formed in a shape in continuous connection with the inside surface of the nozzle member **(31)**, as shown in Figure **33(A)**. On the other hand, in a state where the entire tip end surface of the nozzle member **(31)** is wet, the gas-liquid interface **(52)** is formed in a shape in continuous connection with the outside surface of the nozzle member **(31),** as shown in Figure **33(B)****.** If the cone shaped gas-liquid interface **(52)** has a small diameter, the amount of spray from the nozzle member **(31)** decreases while on the other hand if the gas-liquid interface **(52)** has a large diameter, the amount of spray from the nozzle member **(31)** increases. That is, if the shape of the gas-liquid interface **(52)** in the tip of the nozzle member **(31)** is not stabilized, this produces the problem that the amount of liquid which is sprayed from the nozzle member **(31)** varies.

The present invention provides, as solutions to the above problems, the following fifteenth to twenty-first aspects.

The present invention provides, as a fifteenth aspect according to any one of the first to fourteenth aspects, an electrostatic spraying device in which the nozzle member **(31)** includes a tip end **(31a)** which has a smaller thickness than the rest of the nozzle member **(31).**

In the fifteenth aspect, the nozzle member **(31)** includes a tip end surface **(31b)** having a smaller thickness than that of the rest thereof other than the tip end **(31a).** In other words, in the tip end surface **(31b)** of the nozzle member **(31),** the difference between the inside and outside diameters is small in comparison with the other portions. As described above, in a state where liquid is being sprayed from the nozzle member **(31)**, the gas-liquid interface **(52)** formed in the tip of the nozzle member **(31)** comes to have a cone shape. The diameter of the gas-liquid interface **(52)** formed in a cone shape varies within a range from the inside to the outside diameter of the tip of the nozzle member **(31)**, depending on, for example, variation in the degree of wetness of the tip end surface **(31b)** of the nozzle member **(31).** Compared with this, in the present aspect, the thickness of the tip end surface **(31b)** of the nozzle member **(31)** is made thin, whereby the difference between the inside and outside diameters of the tip end surface **(31b)** is also reduced. Therefore, in accordance with the present aspect, the range of variation in the diameter of the gas-liquid interface **(52)** formed in the tip of the nozzle member **(31)** diminishes.

The present invention provides, as a sixteenth aspect according to any of the first to fourteenth aspects, an electrostatic spraying device in which the thickness of the tip end **(31a)** of the nozzle member **(31)** gradually becomes reduced towards the tip of the nozzle member **(31).**

In the sixteenth aspect, the difference between the inside and outside diameters of the tip end **(31a)** of the nozzle member **(31)** decreases towards the tip of the nozzle member **(31)** and becomes minimized in the endmost portion of the nozzle member **(31).**

The present invention provides, as a seventeenth aspect according to the sixteenth aspect, an electrostatic spraying device in which the inside diameter of the tip end **(31a)** of the nozzle member **(31)** is constant while on the other hand the outside diameter thereof gradually becomes reduced towards the tip of the nozzle member **(31).**

In the seventeenth aspect, the tip end **(31a)** of the nozzle member **(31)** is formed in such a shape that the inside diameter is constant without variation while on the other hand the outside diameter gradually becomes smaller towards the tip. And the difference between the inside and outside diameters of the tip end **(31a)** of the nozzle member **(31)** becomes minimized in the endmost portion of the nozzle member **(31).**

The present invention provides, as an eighteenth aspect according to any one of the first to fourteenth aspects, an electrostatic spraying device in which the nozzle member **(31)** includes a tip end surface **(31b)** which has been treated to become hydrophilic.

In the eighteenth aspect, the tip end surface **(31b)** of the nozzle member **(31)** is treated such that it becomes hydrophilic. Therefore, almost without fail, the entire tip end surface **(31b)** is placed in a wet state. Accordingly, the gas-liquid interface **(52)** formed in the tip of the nozzle member **(31)** has a cone shape in continuous connection with the outside surface of the nozzle member **(31)** (see Figure **33(B)****).** And the diameter of the gas-liquid interface **(52)** is held in approximately the same state as the outside diameter of the nozzle member **(31)** and will change little.

The present invention provides, as a nineteenth aspect according to the eighteenth aspect, an electrostatic spraying device in which the tip end surface **(31b)** of the nozzle member **(31)** has been subjected to a surface roughening process, as a hydrophilic treatment, for surface-roughening the tip end surface **(31b).**

In the nineteenth aspect, the tip end surface **(31b)** of the nozzle member **(31)** is treated such that its surface is roughened. Therefore, almost without fail, the entire tip end surface **(31b)** enters a wet state.

The present invention provides, as a twentieth aspect according to the eighteenth aspect, an electrostatic spraying device in which the tip end surface **(31b)** of the nozzle member **(31)** has been subjected to a process, as a hydrophilic treatment, for forming a groove **(19)** extending in the radial direction of the tip end surface **(31b).**

In the twentieth aspect, at the time when the cone shaped gas-liquid interface **(52)** is formed in the tip of the nozzle member **(31)**, liquid is circulated throughout the groove **(19)** by capillary phenomenon. Consequently, the entire tip end surface **(31b)** of the nozzle member **(31)** is liable to enter a wet state.

The present invention provides, as a twenty-first aspect according to the eighteenth aspect, an electrostatic spraying device in which the tip end surface **(31b)** of the nozzle member **(31)** is subjected to a coating process, as a hydrophilic treatment, for forming a coating composed of a hydrophilic material on the tip end surface **(31b).**

In the twenty-first aspect, owing to the hydrophilic coating formed on the tip end surface **(31b)** of the nozzle member **(31)**, the entire tip end surface **(31b)** of the nozzle member **(31)** is placed in a wet state, almost without fail.

The present invention provides, as a twenty-second aspect according to any one of the first to fourteenth aspects, an electrostatic spraying device in which the tip end surface **(31b)** of the nozzle member **(31)** has been subjected to a water-repellent treatment.

In the twenty-second aspect, since the tip end surface **(31b)** of the nozzle member **(31)** is treated such that it becomes water-repellent, the entire tip end surface **(31b)** is placed in an unwetted state (i.e., dry state), almost without fail. Accordingly, the gas-liquid interface **(52)** formed in the tip of the nozzle member **(31)** has a cone shape in continuous connection with the inside surface of the nozzle member **(31)** (see Figure **33(A)****)**. And the diameter of the gas-liquid interface **(52)** is held in approximately the same state as the inside diameter of the nozzle member **(31)** and will change little.

### ADVANTAGEOUS EFFECTS OF THE INVENTION

In the present invention, the tip of the nozzle member **(31)** is disposed below the liquid level **(51)** in the reservoir member **(20)** to thereby create a head difference between the tip of the nozzle member **(31)** and the liquid level **(51)** in the reservoir member **(20)**, which head difference allows liquid in the reservoir member **(20)** to be supplied to the tip of the nozzle member **(31).** Consequently, in accordance with the present invention, liquid is supplied from within the reservoir member **(20)** to the tip of the nozzle member **(31)** without employing an extruding mechanism using a pump, an electric motor et cetera. Therefore, in accordance with the present invention, it becomes possible to omit the provision of a pump or some like device for feeding liquid to the tip of the nozzle member **(31)** from within the reservoir member **(20),** whereby the electrostatic spraying device **(10)** has a simplified configuration and the cost of production thereof is cut down.

In a conventional electrostatic spraying device in which liquid is supplied to the nozzle by use of a pump or the like, it becomes impossible to atomize liquid in the event of failure of the pump. On the other hand, in accordance with the present invention, there is no need to provide a component member such as a pump for the supply of liquid. This means that the electrostatic spraying device **(10)** no longer looses its functions due to failure of such a component member, thereby making it possible to enhance the reliability of the electrostatic spraying device **(10).**

In the second aspect, when the first electrode **(31, 37)** and the second electrode **(35, 38, 60, 61)** have the same electric potential thereby providing a balance between liquid pressure and surface tension at the gas-liquid interface **(52)** formed in the tip of the nozzle member **(31),** and outflow of the liquid from the reservoir member **(20)** is prevented. Consequently, in the electrostatic spraying device **(10)** of the present aspect which utilizes a head difference for supplying liquid to the nozzle member **(31)** from the reservoir member **(20),** it becomes possible to stop outflow of the liquid from the nozzle member **(31)** by just stopping creating an electric potential difference between the first electrode **(31, 37)** and the second electrode **(35, 38, 60, 61).** Therefore, in accordance with the present aspect, there is no need to provide a configuration for preventing liquid leakage from the nozzle member **(31)** during suspension of the operation of the electrostatic spraying device **(10),** thereby making it possible to simplify the configuration of the electrostatic spraying device **(10)** to a further extent.

In the third aspect, even when nearly all the liquid in the reservoir member **(20)** flows out therefrom, the tip of the nozzle member **(31)** is kept filled with liquid. Therefore, in accordance with the present aspect, it becomes possible to spray almost all of the liquid held in the reservoir member **(20)** from the nozzle member **(31).**

If the nozzle member **(31)** continues spray of the liquid, the height of liquid level **(51)** in the reservoir member **(20)** decreases. If, in the electrostatic spraying device **(10)** of the present aspect, the height of the liquid level **(51)** in the reservoir member **(20)** varies, this causes variation in the head difference between the tip of the nozzle member **(31)** and the liquid level **(51)** in the reservoir member **(20)**. Consequently, if no measurements are taken, the amount of liquid flowing into the nozzle member **(31)** from the reservoir member **(20)** varies and, as a result, the amount of spray from the tip of the nozzle member **(31)** may vary.

On the other hand, in the fourth aspect, the controller means **(17)** controls, in response to variation in the height of the liquid level **(51)** in the reservoir member **(20)**, the duty ratio of the length of time for which the power supply **(16)** is turned on (ON time) and the length of time for which the power supply **(16)** is turned off (OFF time). Therefore, if the duty ratio of the ON and OFF times of the power supply **(16)** is increased in conjunction with decrease in the height of the liquid level **(51)** in the reservoir member **(20),** this makes it possible to maintain the amount of spray from the nozzle member **(31)** constant even when the height of the liquid level **(51)** in the reservoir member **(20)** undergoes a variation.

In the sixth and seventh aspects, an electric field is formed between the head end **(65)** of the second electrode **(60, 61)** and the first electrode **(31,37).** In other words, in the present aspect, the electric field is formed between these relatively narrow regions. And in a space in the vicinity of the tip of the nozzle member **(31),** the electric field is concentrated in a relatively narrow region defined between the head end **(65)** of the second electrode **(60, 61)** and the first electrode **(31**, **37).** Therefore, in accordance with these aspects, it is possible to concentrate an electric field, formed in the vicinity of the tip of the nozzle member **(31),** in a narrow region, whereby, even when the electric field is somewhat disturbed due to an external cause, it is still possible to stably continue spray from the tip of the nozzle member **(31).**

In the eighth aspect, in order to avoid the occurrence of a corona discharge between the first electrode **(31, 37)** and the second electrode **(35, 38, 60, 61),** it is designed such that the value of the electric potential difference created between the first electrode **(31, 37)** and the second electrode **(35, 38, 60, 61)** falls within a predetermined range. If corona discharges are produced in the atmosphere, this gives rise to the possibility of producing ozone harmful to human health. On the other hand, in accordance with the present aspect, since it is arranged such that the electric potential difference between the first electrode **(31, 37)** and the second electrode **(35, 38, 60, 61)** falls within a predetermined range, this ensures avoidance of the production of ozone caused by corona discharge.

In the ninth aspect, the nozzle member **(31)** serves also as the first electrode **(31, 37).** Therefore, in accordance with the present aspect, the number of component parts in the electrostatic spraying device **(10)** can be reduced, and the electrostatic spraying device **(10)** has a further simplified configuration and the cost of production thereof is cut down.

In accordance with each of the tenth, eleventh, twelfth, and fourteenth aspects, the positional relationship between the nozzle member **(31)** and the second electrode **(35, 38, 60, 61)** and the shape of the nozzle member **(31)** can be appropriately set, whereby the electrostatic spraying device **(10)** realized by these aspects is able to stably spray liquid from the nozzle member **(31).**

In the thirteenth aspect, the second electrode **(35, 38, 60, 61)** lies posterior to the tip of the nozzle member **(31),** thereby preventing adhesion of liquid sprayed from the tip of the nozzle member **(31)** to the second electrode **(35, 38, 60, 61)**. If the second electrode **(35, 38, 60, 61)** is contaminated by liquid adhesion thereonto, the electric field formed between the second electrode **(35, 38, 60, 61)** and the first electrode **(31, 37)** becomes unstable, and it may become impossible for the nozzle member **(31)** to continue stable spray therefrom. On the other hand, in accordance with the present aspect, it is possible to continue to form a stable electric field between the second electrode **(35, 38, 60, 61)** and the first electrode **(31, 37)** because the second electrode **(35, 38, 60, 61)** can be kept clean, thereby enabling the nozzle member **(31)** to continue stable liquid spray therefrom.

In each of the fifteenth to seventeenth aspects, the difference between the inside and outside diameters of the tip end surface **(31b)** of the nozzle member **(31)** is made small. As described above, the diameter of the cone shaped gas-liquid interface **(52)** which is formed in the tip of the nozzle member **(31)** during spray operation varies within a range from the inside to the outside diameter of the tip end surface **(31b)** of the nozzle member **(31).** Therefore, if the difference between the inside and outside diameters of the tip end surface **(31b)** of the nozzle member **(31)** is reduced, as in these aspects, this reduces the range of variation in the size of the gas-liquid interface **(52)** formed in the tip of the nozzle member **(31)**. Therefore, in accordance with these aspects, it becomes possible to reduce the range of variation in the amount of liquid which is sprayed from the nozzle member **(31),** thereby eliminating flaws including, for example, unnecessary liquid consumption resulting from excess liquid atomization, insufficiency in the amount of spray, et cetera.

In addition, in the eighteenth aspect, the tip end surface **(31b)** of the nozzle member **(31)** is treated such that it becomes hydrophilic, whereby the entire tip end surface **(31b)** of the nozzle member **(31)** is placed in a wet state during spray operation. And the cone shaped gas-liquid interface **(52)** formed in the tip of the nozzle member **(31)** has a shape in continuous connection with the outside surface of the nozzle member **(31),** as shown in Figure **33(B)** and stably remains in such a shape. Therefore, in accordance with the present aspect, it becomes possible to inhibit variation in the size of the gas-liquid interface **(52)** formed in the tip of the nozzle member **(31),** whereby the amount of liquid which is sprayed from the nozzle member **(31)** is held approximately constant.

In addition, in the twenty-second aspect, the tip end surface **(31b)** of the nozzle member **(31)** is treated such that it becomes water-repellent, whereby the entire tip end surface **(31b)** of the nozzle member **(31)** is placed in an unwetted state during spray operation. And the cone shaped gas-liquid interface **(52)** formed in the tip of the nozzle member **(31)** has a shape in continuous connection with the inside surface of the nozzle member **(31),** as shown in Figure **33(A)** and stably remains in such a shape. Therefore, in accordance with the present aspect, it becomes possible to inhibit variation in the size of the gas-liquid interface **(52)** formed in the tip of the nozzle member **(31)**, whereby the amount of liquid which is sprayed from the nozzle member **(31)** is held approximately constant.

### BRIEF DESCRIPTION OF THE DRAWINGS

In the drawings:
Figure **1** is a schematic block diagram of an electrostatic spraying device according to a first embodiment of the present invention;
Figure **2** is a perspective view of a spray cartridge in the first embodiment;
Figure **3** is an exploded perspective view of the spray cartridge of the first embodiment;
Figure **4** is a cross sectional view showing a chief part of the spray cartridge in the first embodiment;
Figure **5** is a schematic front view of a chief part of the spray cartridge in the first embodiment, representing a positional relationship between a spray nozzle and a ring electrode;
Figure **6(A)** is a cross sectional view diagrammatically showing the tip of the spray nozzle during spray operation and Figure **6(B)** is a cross sectional view diagrammatically showing the tip of the spray nozzle during suspension of the spray operation;
Figure **7** is a time chart showing the operation of a controller in the first embodiment;
Figure **8** is a table showing results of spray tests performed on spray nozzles of different thicknesses;
Figure **9** is a table showing results of spray tests performed on spray nozzles of different inside diameters;
Figure **10** is a table showing results of spray tests performed on spray cartridges of different horizontal inter-electrode distances;
Figure **11** is a schematic front view of a chief part of a spray cartridge in a variation of the first embodiment, showing a positional relationship between a spray nozzle and a ring electrode;
Figure **12** is a schematic front view of a chief part of a spray cartridge in another variation of the first embodiment, showing a positional relationship between a spray nozzle and a ring electrode;
Figure **13** is a schematic block diagram of an electrostatic spraying device according to a second embodiment of the present invention;
Figure **14** is a perspective view of a spray cartridge in the second embodiment;
Figure **15** is a cross sectional view showing a chief part of the spray cartridge of the second embodiment;
Figure **16** is a schematic front view of a chief part of the spray cartridge in the second embodiment, representing a positional relationship between a spray nozzle and a rod-shaped electrode;
Figure **17** is a schematic front view of a chief part of a spray cartridge in a variation of the second embodiment, representing a positional relationship between a spray nozzle and a rod-shaped electrode;
Figure **18** is a cross sectional view diagrammatically showing the tip of a spray nozzle in a third embodiment of the present invention;
Figure **19** is a cross sectional view diagrammatically showing the tip of the spray nozzle during spray operation in the third embodiment;
Figure **20** is a relational diagram showing a relationship between the vertex angle, θ, of the tip end of a spray nozzle and the variation in the amount of spray;
Figure **21** is a cross sectional view diagrammatically showing the tip of a spray nozzle in a first variation of the third embodiment;
Figure **22** is a cross sectional view diagrammatically showing the tip of a spray nozzle during spray operation in a second variation of the third embodiment;
Figure **23** is a cross sectional view of the tip end of a spray nozzle in a third variation of the third embodiment;
Figure **24** is a cross sectional view of the tip end of another spray nozzle in the third variation of the third embodiment;
Figure **25** is a schematic perspective view of the tip end of a spray nozzle in a fourth embodiment of the present invention;
Figure **26** is a cross sectional view diagrammatically showing the tip of the spray nozzle during spray operation in the fourth embodiment;
Figure **27** is a cross sectional view diagrammatically showing the tip of a spray nozzle during spray operation in a second variation of the fourth embodiment;
Figure **28** is a cross sectional view diagrammatically showing the tip of a spray nozzle during spray operation in a fifth embodiment of the present invention;
Figure **29** is a schematic cross sectional view of a spray cartridge in a first variation of another embodiment of the present invention;
Figure **30** is a schematic cross sectional view of another spray cartridge in the first variation of the other embodiment;
Figure **31** is a schematic perspective view of an air cleaning device to which mounted is an electrostatic spraying device in a third variation of the other embodiment;
Figure **32** is a cross sectional view showing a chief part of a spray cartridge in a fourth variation of the other embodiment; and
Figure **33**, comprised of Figure **33(A)** and Figure **33(B)**, is a chief part cross sectional view of a spray nozzle showing the shape of a gas-liquid interface formed in the tip of the spray nozzle, wherein Figure **33(A)** depicts a state where the entire tip end surface of the spray nozzle is not wet while on the other hand Figure **33(B)** depicts a state where the entire tip end surface of the spray nozzle is wet.

### REFERENCE NUMERALS IN THE DRAWINGS

- 10: ELECTROSTATIC SPRAYING DEVICE
- 15: SPRAY CARTRIDGE
- 16: POWER SUPPLY
- 17: CONTROLLER PART (CONTROLLER MEANS)
- 20: SOLUTION TANK (RESERVOIR MEMBER)
- 31: SPRAY NOZZLE (NOZZLE MEMBER, FIRST ELECTRODE)
- 31a: TIP END
- 31b: TIP END SURFACE
- 35: RING ELECTRODE (SECOND ELECTRODE)
- 37: NEEDLE ELECTRODE (FIRST ELECTRODE)
- 38: ELECTRODE MEMBER (SECOND ELECTRODE)
- 51: LIQUID LEVEL
- 60: ROD-SHAPED ELECTRODE (SECOND ELECTRODE)
- 61: TRIANGULAR PLATE ELECTRODE (SECOND ELECTRODE)
- 65: HEAD END
- 71: HYDROPHILIC COATING
- 72: WATER-REPELLENT COATING

### BEST EMBODIMENT MODE FOR CARRYING OUT THE INVENTION

Hereinafter, preferred embodiments of the present invention will be described in detail with reference to the accompanying drawings.

### FIRST EMBODIMENT OF THE INVENTION

Description will be made in regard to the invention.

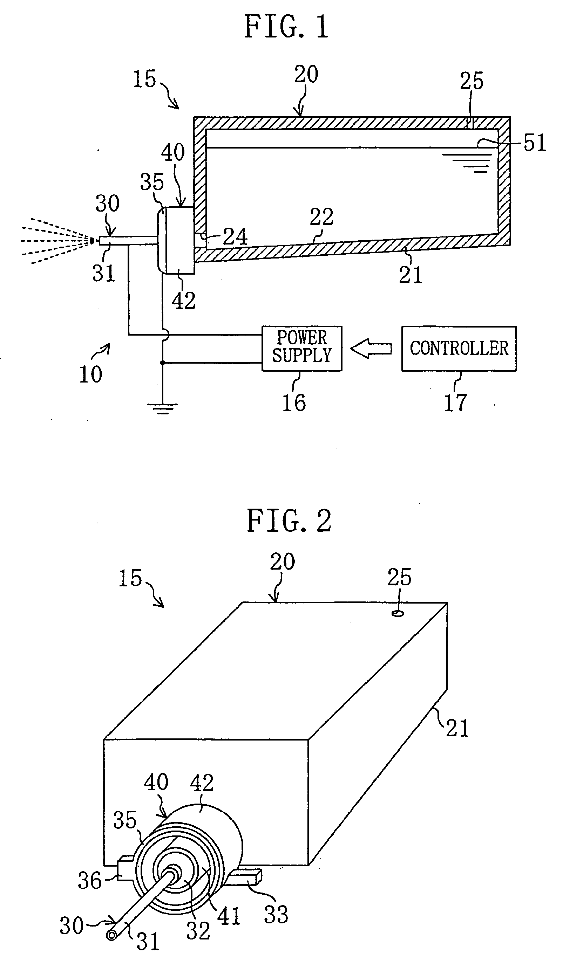

Referring first to Figure 1, there is shown an electrostatic spraying device **(10)** of the present embodiment. The electrostatic spraying device **(10)** has a spray cartridge **(15)**, a power supply **(16)**, and a controller **(17)**.

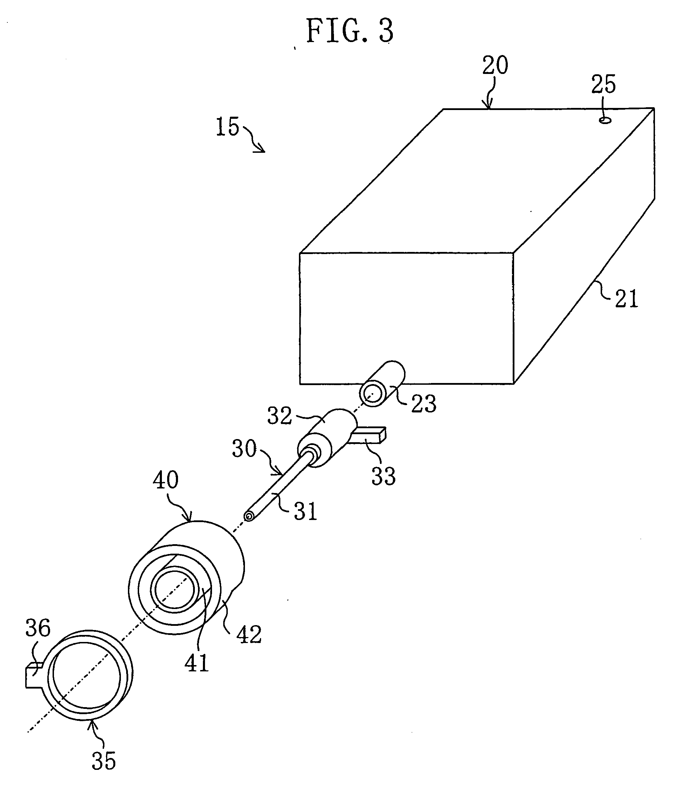

As shown in Figures 2 and 3, the spray cartridge **(15)** has a solution tank **(20),** a nozzle unit **(30),** an electrode holder **(40),** and a ring electrode **(35).**

The solution tank **(20)** constitutes a reservoir member and has a tank body **(21).** The tank body **(21)** is a hollow reservoir which is formed in a somewhat flat, rectangular parallelepiped shape. The tank body **(21)** is provided, in its top plate, with an air purge hole **(25).** The tank body **(21)** has a bottom surface **(22).** The bottom surface **(22)** is an inclined surface inclining from the back surface (right side surface in Figure **1**; side surface on the far side in Figures **2** and **3**) of the tank body **(21)** towards the front surface (left side surface in Figure **1**; side surface on the near side in Figures **2** and **3****)** of the tank body **(21)**. And the tank body **(21)** has a deeper depth on the front surface side than that on the back surface side. In addition, the side surfaces of the tank body **(21)** are approximately vertical surfaces.

Mounted on the front surface of the tank body **(21)** is a pipe part **(23)**. The pipe part **(23)** is formed in a relatively short tubular shape, and projects from the front surface of the tank body **(21)** in a substantially horizontal direction. The pipe part **(23)** is mounted on the front surface of the tank body **(21)** such that it is arranged near the lower end of the front surface and lies substantially centrally (relative to the width direction) in the front surface. In addition, a through hole **(24)** is formed through a wall constituting the front surface of the tank body **(21).** The internal space of the tank body **(21)** and the pipe part **(23)** fluidly communicate with each other via the through hole **(24)**. The lower end of the through hole **(24)** is situated slightly above the bottom surface **(22)** of the tank body **(21)** (see Figure **1**).

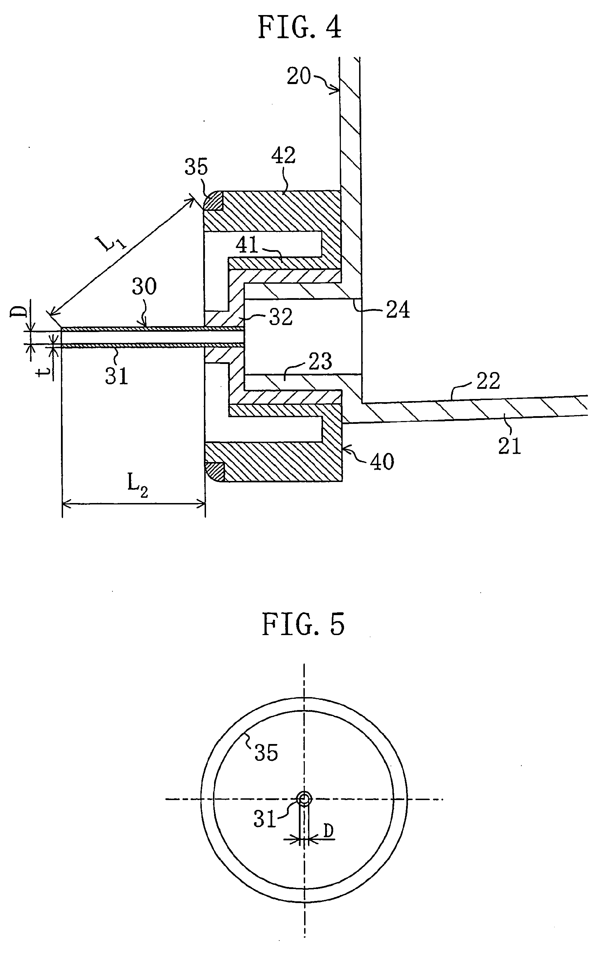

As shown in Figures **3** and **4**, the nozzle unit **(30)** has a spray nozzle **(31)** and a nozzle holder **(32).**

The spray nozzle **(31)** is a circular tube of stainless steel, and constitutes a nozzle member. On the other hand, the nozzle holder **(32)** is shaped like a cylindrical cap with a bottom. The nozzle holder **(32)** has an inside diameter substantially equal to the outside diameter of the pipe part **(23)**, and is placed over the pip part **(23).** In other words, the pip part **(23)** of the solution tank **(20)** is inserted into the nozzle holder **(32)**. The base end of the spray nozzle **(31)** is inserted into the center of the bottom (end on the near side in Figure 3) of the nozzle holder **(32)**. The base end of the spray nozzle **(31)** passes completely through the bottom of the nozzle holder **(32).** In the state where the nozzle unit **(30)** is mounted to the solution tank **(20),** the spray nozzle **(31)** projects from the front surface of the tank body **(21)** in a substantially horizontal direction and fluidly communicates, through the pipe part **(23)** and the through hole **(24),** with the internal space of the tank body **(21).**

The nozzle holder **(32)** has a terminal part **(33).** The terminal part **(33)** projects from the outer peripheral surface of the nozzle holder **(32),** and is arranged on the open end side (far side in Figure **3**) of the nozzle holder **(32)**. The entire nozzle holder **(32)** including the terminal part **(33)** is formed of an electrically conducting resin. And the spray nozzle **(31)** inserted into the bottom of the nozzle holder **(32)** is electrically connected to the nozzle holder **(32)**, and constitutes a first electrode.

The electrode holder **(40)** has an inner tubular part **(41)** and an outer tubular part **(42)**. The inner tubular part **(41)** and the outer tubular part **(42)** are circular tubes. The outer tubular part **(42)** has an inside diameter greater than the outside diameter of the inner tubular part **(41)**. The inner tubular part **(41)** and the outer tubular part **(42)** are arranged coaxially with each other and coupled together at their base ends to be integrated. The inner tubular part **(41)** has an inside diameter approximately equal to the outside diameter of the nozzle holder **(32).** The electrode holder **(40)** is mounted to the nozzle holder **(32)** by engagement between the inner tubular part **(41)** and the nozzle holder **(32)**, with the base ends of the inner and outer tubular parts **(41, 42)** oriented towards the tank body **(21)** of the solution tank **(20).** The entire electrode holder **(40)** is formed of a non electrically conducting resin.

The ring electrode **(35)** is formed in a circular ring shape. The ring electrode **(35)** has a terminal part **(36).** The terminal part **(36)** projects outwardly from the outer peripheral surface of the ring electrode **(35)** in the radial direction thereof. The entire ring electrode **(35)** including the terminal part **(36)** is formed of an electrically conducing resin, and constitutes a second electrode. The ring electrode **(35)** is mounted to the outer tubular part **(42)** of the electrode holder **(40).** More specifically, the ring electrode **(35)** is engaged onto the outer circumferential edge of the outer tubular part **(42)** on the side of the tip thereof. As described above, the electrode holder **(40)** is formed of non electrically conducting resin. Accordingly, the ring electrode **(35)** is electrically insulated from the spray nozzle **(31).**

The aforesaid materials used to form the spray nozzle **(31)** and the ring electrode **(35)** are taken just as exemplary ones. That is, the spray nozzle **(31)** may be formed of an electrically conducting material (for example, electrically conducting resin) other than stainless steel. In addition, the ring electrode **(35)** may be formed of an electrically conducting material (for example, metal) other than electrically conducing resin.

The tank body **(21)** of the solution tank **(20)** contains therein a water solution of a substance beneficial to the human body, e.g., a water solution of theanine which is a kind of amino acid. The position of the liquid level **(51)** in the tank body **(21)** is higher than the tip of the spray nozzle **(31)** extending horizontally from the lower part of the tank body **(21).** There is a head difference between the liquid level **(51)** in the tank body **(21)** and the tip of the spray nozzle **(31)** and, because of such a head difference, the water solution in the tank body **(21)** is supplied to the tip of the spray nozzle **(31)**.

Referring to Figures **4** and **5**, the detailed shape of the spray nozzle **(31)** constituting a first electrode and the positional relationship between the spray nozzle **(31)** and the ring electrode **(35)** constituting a second electrode are described. The spray nozzle **(31)** has an outside diameter of 0.7 mm and an inside diameter (D) of 0.4 mm. The spray nozzle **(31)** has a thickness (t) of 0.15 mm. The thickness of the spray nozzle **(31)** is constant over its full length. The direct distance from the outer circumferential edge of the tip of the spray nozzle **(31)** to the front surface of the ring electrode **(35)**, i.e., the distance (L₁) between the tip of the spray nozzle **(31)** and the ring electrode **(35),** is 5.0 mm. In addition, the ring electrode **(35)** is arranged nearer to the tank body **(21)** than the tip of the spray nozzle **(31)**. The distance between the tip end surface of the spray nozzle **(31)** and the front surface of the ring electrode **(35),** i.e., the horizontal distance (L₂) between the tip of the spray nozzle **(31)** and the ring electrode **(35)** is 5.0 mm.

The power supply **(16)** is a direct-current high-voltage power supply. The power supply **(16)** is electrically connected, at its positive terminal, to the spray nozzle **(31)** through the terminal part **(33)** of the nozzle holder **(32)** while the negative terminal thereof is electrically connected to the terminal part **(36)** of the ring electrode **(35).** The negative terminal of the power supply **(16)** is connected to ground (earthed). When the power supply **(16)** is turned on, a voltage of about 6 kV is applied to the spray nozzle **(31).** Figure **1** omits diagrammatical representation of the terminal part **(33)** and therefore shows, for the sake of simplicity, an arrangement in which the power supply **(16)** is connected to the spray nozzle **(31).**

The controller **(17)** performs switching of the power supply **(16)** and constitutes a controller means. More specifically, the controller **(17)** is configured such that the power supply **(16)** alternately repeats switching between the ON and OFF states. In addition, the controller **(17)** is configured such that it controls, in response to variation in the height of the liquid level **(51)** in the solution tank **(20),** the ratio between the length of time (ON time) for which the power supply **(16)** is turned on and the length of time (OFF time) for which the power supply **(16)** is turned off, i.e., the duty ratio.

### RUNNING OPERATION

Description will be made in regard to the running operation of the electrostatic spraying device **(10)**. The electrostatic spraying device **(10)** performs EHD spay in a so-called cone jet mode.

As described above, in the spray cartridge **(15)**, the liquid level **(51)** in the solution tank **(20)** lies above the tip of the spray nozzle **(31)** and, as a result, there is a head difference between the liquid level **(51)** in the solution tank **(20)** and the tip of the spray nozzle **(31).** Consequently, a liquid pressure caused by the head difference acts on the gas-liquid interface **(52)** formed in the tip of the spray nozzle **(31).**

In a state where the power supply **(16)** is placed in the OFF state (i.e., when the spray nozzle **(31)** and the ring electrode **(35)** have the same electric potential), this state provides a balance between surface tension and liquid pressure (caused by the head difference) of the water solution in the gas-liquid interface **(52)** formed in the tip of the spray nozzle **(31)**. Consequently, no water solution flows out from the tip of the spray nozzle **(31)** even when the power supply **(16)** is placed in the OFF state. In the electrostatic spraying device **(10)** of the present embodiment which employs the spray nozzle **(31)** whose inside diameter is 0.4 mm, leakage of the water solution from the tip of the spray nozzle **(31)** is prevented even when a liquid pressure of 20 mmH₂O acts on the gas-liquid interface **(52)** in the tip of the spray nozzle **(31)**, when the concentration of the water solution of theanine is at 10% by mass.

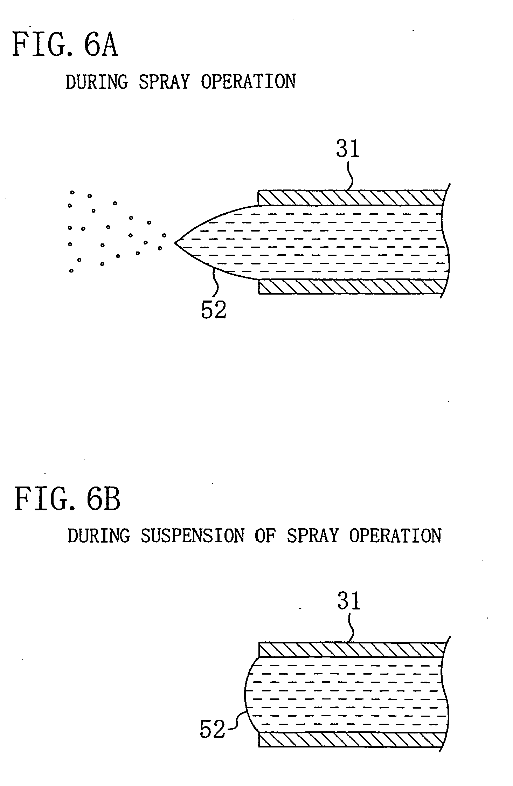

In a state where the power supply **(16)** is placed in the ON state (i.e., when there is created an electric potential difference between the spray nozzle **(31)** and the ring electrode **(35))**, an electric field is formed in the vicinity of the tip of the spray nozzle **(31)**. In addition, the water solution in the spray nozzle **(31)** polarizes, and plus electric charges are collected in the vicinity of the gas-liquid interface **(52)** in the tip of the spray nozzle **(31).** And in the tip of the spray nozzle **(31)**, as shown in Figure **6(A)**, the gas-liquid interface **(52)** is extended to have a cone shape, and a part of the water solution is "tore off" from the top of the cone-shaped gas-liquid interface **(52)** to become liquid droplets.

The spray nozzle **(31)** discharges, at its tip, minute liquid droplets of the theanine water solution, and these liquid droplets are supplied to the room air. A user staying in the room inhales liquid droplets of the theanine water solution present in the air together with air when taking his/her breath. The liquid droplets inhaled by the user adhere to the alveolar mucosa. Theanine contained in the liquid droplets is taken into the user's body through the alveolar mucosa. Theanine allegedly has efficacy to keep down excitation to thereby induce relaxation.

In order that liquid droplets contained in the inhaled air may reach the user's alveolar mucosa, the diameter of the liquid droplets should fall within a predetermined range. If the liquid droplet diameter is too large, liquid droplets inhaled by the user are trapped by the airway mucosa and fail to reach the alveolar mucosa. On the other hand, if the liquid droplet diameter is too small, liquid droplets inhaled by the user are discharged together with inhaled air, without being trapped by the alveolar mucosa. In consideration of these conditions, the diameter of liquid droplets which are sprayed from the electrostatic spraying device **(10)** is preferably not less than 10 nm nor more than 5 µm, more preferably not less than 50 nm nor more than 2 µm.

As described above, in the spray cartridge **(15)**, water solution in the form of liquid droplets is discharged from the tip of the spray nozzle **(31).** Consequently, if water solution is not supplied into the spray nozzle **(31)**, the amount of water solution in the spray nozzle **(31)** decreases and it becomes impossible to continue spray. On the other hand, in the spray cartridge **(15)**, the tip of the spray nozzle **(31)** lies at a position below the liquid level **(51)** in the solution tank **(20),** in other words, there is a head difference between the liquid level **(51)** in the solution tank **(20)** and the tip of the spray nozzle **(31)**. Owing to such a head difference, the water solution in the solution tank **(20)** is supplied into the spray nozzle **(31)**, and spray from the spray nozzle **(31)** is continuously performed. That is to say, in the electrostatic spraying device **(10)** of the present embodiment, there is no need to provide a component member such as a pump for supplying the water solution in the solution tank **(20)** to the spray nozzle **(31)**.

### OPERATION OF THE CONTROLLER

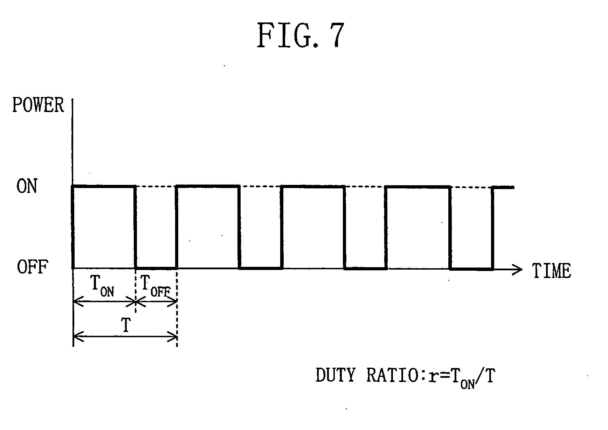

The controller **(17)** controls the power supply **(16)** such that the power supply **(16)** is alternately repeatedly switched between the ON and OFF states in a constant cycle. This operation of the controller **(17)** will be described with reference to Figure 7.

The controller **(17)** places the power supply **(16)** in the OFF state upon an elapse of time (T_{ON}) since the time when the power supply **(16)** was turned on. Thereafter, the controller **(17)** holds the power supply **(16)** in the OFF state. Then, upon an elapse of time (T_{OFF}) since the time when the power supply (16) is turned off, the controller **(17)** again places the power supply **(16)** in the ON state. The controller **(17)** periodically repeats this operation. The cycle of operation that the controller **(17)** performs, i.e., the sum (T) of the ON time (T_{ON}) and OFF time (T_{OFF}) of the power supply **(16)** (T = T_{ON} + T_{OFF}), is constant.

If, in the spray cartridge **(15),** spray is continued, the liquid level **(51)** in the solution tank **(20)** is gradually lowered. In the spray cartridge **(15)**, the head difference between the tip of the spray nozzle **(31)** and the liquid level **(51)** in the solution tank **(20)** varies as the height of the liquid level **(51)** in the solution tank **(20)** varies. Consequently, if no measures are taken, the volume of flow of the water solution towards the spray nozzle **(31)** varies, in association with which the amount of spray from the spray nozzle **(31)** varies.

In view of the above, the controller **(17)** controls, in response to variation in the height of the liquid level **(51)** in the solution tank **(20)**, the duty ratio, r (= T_{ON}/T), of the ON and OFF times of the power supply **(16)**. More specifically, the controller **(17)** increases the duty ratio (r) as the liquid level **(51)** in the solution tank **(20)** becomes lower. Stated another way, the controller **(17)** gradually increases the ratio of the ON time (T_{ON}) of the power supply **(16)** to the cycle (T) of the ON/OFF of the power supply **(16)** by the controller **(17),** thereby prolonging the length of time for which the power supply **(16)** is placed in the ON state to cause liquid droplets to be discharged from the spray nozzle **(31).**

As described above, when the volume of flow of the water solution towards the spray nozzle **(31)** from the solution tank **(20)** decreases as the liquid level **(51)** in the solution tank **(20)** becomes lower, the controller **(17)** accordingly increases the duty ratio (r) to prolong the length of time for which spray is performed in the spray cartridge **(15)**. At the same time, the controller **(17)** controls the duty ratio (r) so that, even when the liquid level **(51)** in the solution tank **(20)** is lowered, the amount of spray from the spray nozzle **(31)** is kept constant.

In order to control the duty ratio (r), the controller **(17)** requires information about the height of the liquid level **(51)** in the solution tank **(20)**. On the other hand, if the amount of spray from the spray nozzle **(31)** is held constant, the speed at which the liquid level **(51)** in the solution tank **(20)** is lowered also becomes constant. In other words, the height of the liquid level **(51)** in the solution tank **(20)** is proportional to the integrated value of the length of time (spray time) for which spray is performed in the spray cartridge **(15)**. Making utilization of such a relationship between the integrated value of the length of spray time and the height of the liquid level **(51)**, the controller **(17)** controls, depending on the integrated value of the length of spray time in the spray cartridge **(15)**, the duty ratio (r).

### DIMENSIONS OF EACH PART OF THE SPRAY CARTRIDGE

In the spray cartridge **(15)**, it is preferable that the thickness of the tip of the spray nozzle **(31)** (length (t) in Figure **4**), the inside diameter of the spray nozzle **(31)** (length (D)), the horizontal distance between the tip of the spray nozzle **(31)** and the ring electrode **(35)** (length (L₂)), and the distance between the tip of the spray nozzle **(31)** and the ring electrode **(35)** (length (L)) are set to fall within the following ranges, respectively.

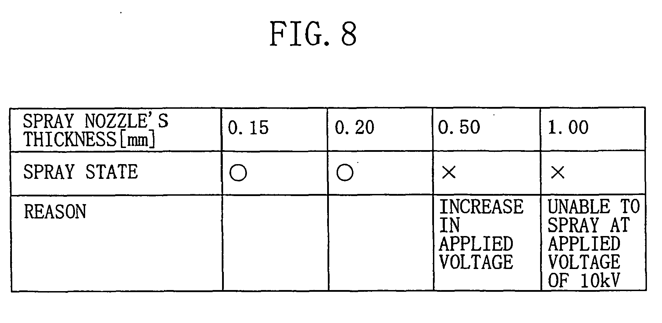

In the first place, description will be made in regard to the thickness (t) of the tip of the spray nozzle **(31)**. In order to stably atomize water solution in the tip of the spray nozzle **(31)**, an electric field is preferably concentrated in the vicinity of an inner-peripheral side edge in the tip end surface of the spray nozzle **(31)**. As shown in Figure 8, if the thickness of the tip of the spray nozzle **(31)** is 0.2 mm or less, atomization of the water solution is stably performed in the tip of the spray nozzle **(31)**. On the other hand, if the thickness of the tip of the spray nozzle **(31)** is 0.5 mm or more, this makes it impossible to stably atomize water solution unless the electric potential difference between the spray nozzle **(31)** and the ring electrode **(35)** is increased. In consideration of these conditions, preferably the tip of the spray nozzle **(31)** has a thickness of 0.2 mm or less.

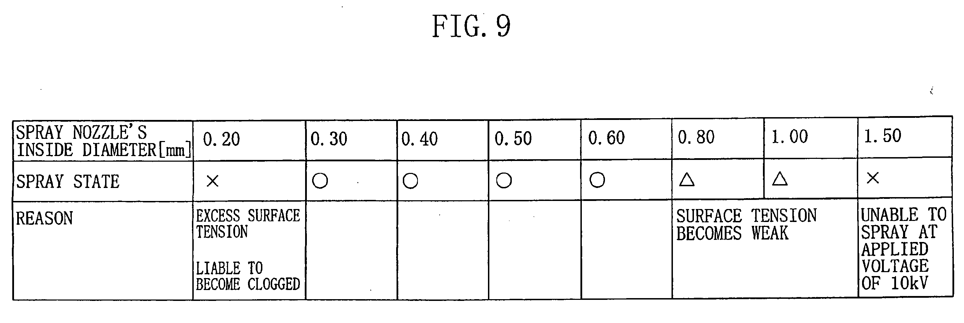

Next, description will be made in regard to the inside diameter (D) of the spray nozzle **(31)**. Surface tension in the gas-liquid interface **(52)** of the tip of the spray nozzle **(31)** is determined depending on the inside diameter of the of the spray nozzle **(31)** in the case of the same value for the degree of viscosity of the water solution. Generally, surface tension in the gas-liquid interface **(52)** increases as the inside diameter of the spray nozzle **(31)** decreases while on the other hand it decreases as the inside diameter of the spray nozzle **(31)** increases. As shown in Figure **9**, if the inside diameter of the spray nozzle **(31)** is 0.2 mm or less, surface tension in the gas-liquid interface **(52)** becomes excessively high, this makes it difficult to stably atomize water solution in the tip of the spray nozzle **(31).** In addition, the spray nozzle **(31)** tends to become clogged, thereby giving rise to the possibility that the reliability of the electrostatic spraying device **(10)** becomes impaired. On the other hand, if the inside diameter of the spray nozzle **(31)** is 1.5 mm or more, surface tension in the gas-liquid interface **(52)** becomes excessively low, this also makes it difficult to stably atomize water solution in the tip of the spray nozzle **(31)**. In consideration of these conditions, preferably the inside diameter (D) of the spray nozzle **(31)** is not less than 0.3 mm nor more than 1.0 mm.

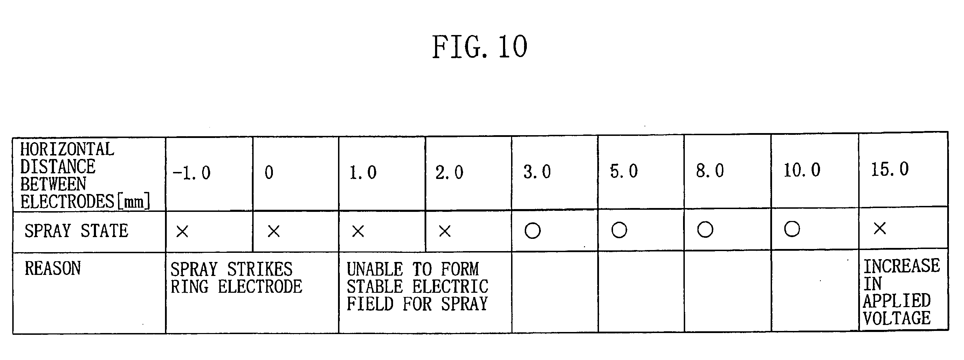

In the second place, description will be made in regard to the horizontal distance between the tip of the spray nozzle **(31)** and the ring electrode **(35)**, i.e., the backed-away distance (L₂) of the ring electrode **(35)** with respect to the tip of the spray nozzle **(31)**. As shown in Figure 10, if the ring electrode **(35)** is arranged ahead of the tip of the spray nozzle **(31)** or in the same place as the tip of the spray nozzle **(31)**, liquid droplets sprayed from the tip of the spray nozzle **(31)** are attracted to the ring electrode **(35)**, thereby making it impossible to supply atomized water solution into the room. In addition, if, even when the ring electrode **(35)** is arranged at a rearward position backed away from the tip of the spray nozzle **(31)**, the backed-away distance (L₂) of the ring electrode **(35)** with respect to the tip of the spray nozzle **(31)** is too short, this makes it difficult to form an electric field suitable for spray in the vicinity of the tip of the spray nozzle **(31)**. Conversely, if the backed-away distance (L₂) of the ring electrode **(35)** with respect to the tip of the spray nozzle **(31)** is too long, this makes it impossible to stably atomize water solution unless the electrical potential difference between the spray nozzle **(31)** and the ring electrode **(35)** is increased. In consideration of these conditions, preferably the horizontal backed-away distance (L₂) of the ring electrode **(35)** with respect to the tip of the spray nozzle **(31)** is not less than 3.0 mm nor more than 10.0 mm.

Finally, description will be made in regard to the distance (L₁) between the tip of the spray nozzle **(31)** and the ring electrode **(35)**. If the distance (L₁) between the tip of the spray nozzle **(31)** and the ring electrode **(35)** is too short, the electric potential difference at which a corona discharge starts occurring between the spray nozzle **(31)** and the ring electrode **(35)** decreases. And if trying to avoid corona discharges in order to suppress the production of ozone, it becomes impossible to create a sufficient electric potential difference between the spray nozzle **(31)** and the ring electrode **(35)**, thereby making it difficult to stably atomize water solution in the tip of the spray nozzle **(31)**. Conversely, if the distance (L₁) between the tip of the spray nozzle **(31)** and the ring electrode **(35)** is too long, this makes it impossible to stably atomize water solution unless the electric potential difference between the spray nozzle **(31)** and the ring electrode **(35)** is increased. In consideration of these conditions, preferably the distance (L₁) between the tip of the spray nozzle **(31)** and the ring electrode **(35)** is not less than 5.0 mm nor more than 20.0 mm.

### ELECTRIC POTENTIAL DIFFERENCE BETWEEN THE SPRAY NOZZLE AND THE RING ELECTRODE

Preferably, the electric potential difference created between the spray nozzle **(31)** and the ring electrode **(35)** falls within the following range.

More specifically, if the electric potential difference between the spray nozzle **(31)** and the ring electrode **(35)** is too small, this makes it impossible to stably atomize water solution in the tip of the spray nozzle **(31)**. Consequently, the electric potential difference created between the spray nozzle **(31)** and the ring electrode **(35)** is preferably 3 kV or more. On the other hand, if the electric potential difference between the spray nozzle **(31)** and the ring electrode **(35)** increases, this may give rise to the possibility of causing a corona discharge. If a corona discharge is produced in the atmosphere, this generates ozone harmful to human health. Consequently, the electric potential difference created between the spray nozzle **(31)** and the ring electrode **(35)** preferably falls below a value at which a corona discharge starts occurring.

The value of the electric potential difference at which a corona discharge starts occurring varies depending on the distance between the spray nozzle **(31)** and the ring electrode **(35)**. In the electrostatic spraying device **(10)** of the present embodiment, if the electric potential difference between the spray nozzle **(31)** and the ring electrode **(35)** is 7 kV or more, this gives rise to the possibility of occurrence of a corona discharge. Accordingly, in the electrostatic spraying device **(10)**, preferably the electric potential difference created between the spray nozzle **(31)** and the ring electrode **(35)** is not less than 3 kV but falls below 7 kV.

### ADVANTAGEOUS EFFECTS OF THE FIRST EMBODIMENT

In the present embodiment, the tip of the spray nozzle **(31)** is arranged below the liquid level **(51)** in the solution tank **(20)** to thereby create a head difference therebetween, which head difference allows the liquid in the solution tank **(20)** to be supplied to the tip of the spray nozzle **(31)**. As a result of such arrangement, it becomes possible to supply liquid to the tip of the spray nozzle **(31)** from within the solution tank **(20)**, without employing an extruding mechanism using a pump, an electric motor et cetera. Therefore, in accordance with the present embodiment, it becomes possible to omit the provision of a pump or some like device for feeding liquid to the tip of the nozzle member **(31)** from within the solution tank **(20)**, whereby the electrostatic spraying device **(10)** has a simplified configuration and the cost of production thereof is cut down.

In a conventional electrostatic spraying device in which liquid is supplied to the nozzle by use of a pump or the like, it becomes impossible to atomize liquid in the event of failure of the pump. On the other hand, in accordance with the electrostatic spraying device **(10)** of the present embodiment, there is no need to provide a component member such as a pump for providing a supply of liquid to the nozzle. Therefore, in accordance with the present embodiment, it becomes possible to reduce the possibility that the electrostatic spraying device **(10)** looses its functions due to the pump or the like breaking down, thereby making it possible to enhance the reliability of the electrostatic spraying device **(10).**

In addition, in the present embodiment, when the spray nozzle **(31)** and the ring electrode **(35)** have the same electric potential, there is established a balance between liquid pressure and surface tension at the gas-liquid interface **(52)** formed in the tip of the nozzle member **(31)**, whereby outflow of the liquid from the spray nozzle **(31)** is prevented. Consequently, in the electrostatic spraying device **(10)** of the present embodiment, it becomes possible to stop liquid outflow from the spray nozzle **(31)** by just turning off the power supply **(16)** to thereby place the spray nozzle **(31)** and the ring electrode **(35)** in the same electric potential state. Therefore, in accordance with the present embodiment, there is no need to provide a configuration designed for preventing liquid leakage from the spray nozzle **(31)** when the electrostatic spraying device **(10)** is stopped, thereby making it possible to simplify the configuration of the electrostatic spraying device **(10)** to a further extent.

In addition, in the present embodiment, the controller **(17)** increases the duty ratio of the ON and OFF times of the power supply **(16)** in order to cope with the lowering of the height of the liquid level **(51)** in the solution tank **(20)** when spray is being continued. Consequently, even when the volume of flow of the water solution towards the spray nozzle **(31)** from the solution tank **(20)** is reduced because the head difference between the tip of the spray nozzle **(31)** and the liquid level **(51)** in the solution tank **(20)** is varied in association with the lowering of the liquid level **(51)** in the solution tank **(20)**, the amount of spray from the spray nozzle **(31)** can be held constant.

In the present embodiment, the ring electrode **(35)** serving as a second electrode is arranged behind the tip of the spray nozzle **(31)**. This arrangement prevents liquid sprayed from the tip of the spray nozzle **(31)** from adhering to the ring electrode **(35).** If the ring electrode **(35)** is contaminated by adhesion of the liquid thereto, this renders the electric field between the ring electrode **(35)** and the spray nozzle **(31)** unstable, thereby giving rise to the possibility that spray from the spray nozzle **(31)** cannot be stably continuously performed. On the other hand, in accordance with the present embodiment, the ring electrode **(35)** is kept clean, thereby making it possible to continuously form a stable electric field between the ring electrode **(35)** and the spray nozzle **(31)**, and spray of the liquid from the spray nozzle **(31)** is stably continued.

### VARIATION OF THE FIRST EMBODIMENT

In the spray cartridge **(15)** of the present embodiment, a plurality of electrode members **(38)** as a second electrode may be provided as a substitute for the ring electrode **(35)**.

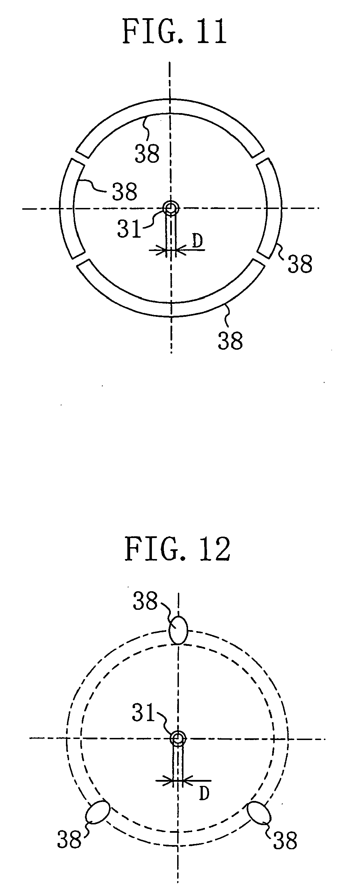

For example, as shown in Figure **11**, the spray cartridge **(15)** may have, as a second electrode, a plurality of electrode members **(38)** each of which is formed into an arc-like, linear shape. In this case, the electrode members **(38)** have the same curvature radius and are arranged on a single pitch circle. The center of the pitch circle of these electrode members **(38)** lies on the central axis of the spray nozzle **(31)**. The distance (L₁) from the inner periphery of each electrode member **(38)** to the outer peripheral surface of the spray nozzle **(31)** is the same as the other electrode member.

In addition, as shown in Figure **12**, the spray cartridge **(15)** may have, as a second electrode, a plurality of electrode members **(38)** each of which is shaped like a small piece having a cross section of oval shape. Also in this case, the electrode members **(38)** are arranged on a single pitch circle. The center of the pitch circle of these electrode members **(38)** lies on the central axis of the spray nozzle **(31)**. The distance (L₁) from the inner periphery of each electrode member **(38)** to the outer peripheral surface of the spray nozzle **(31)** is the same as the other electrode member.

As described above, in the spray cartridge **(15)** of each variation, a plurality of electrode members **(38)** are provided as a second electrode. These electrode members **(38)** may be arranged on a pitch circle concentric with the spray nozzle **(31)**. In this case, the pitch circle of the plural electrode members **(38)** has a radius which is approximately the same as that of the ring electrode **(35)** of the first embodiment. In addition, preferably the backed-away distance of the pitch circle from the tip of the spray nozzle **(31)** approximately equals the backed-away distance of the ring electrode **(35)**.

### SECOND EMBODIMENT OF THE PRESENT INVENTION

Description will be made in regard to a second embodiment of the present invention. In regard to the electrostatic spraying device **(10)** of the present embodiment, the difference from the first embodiment is described below.

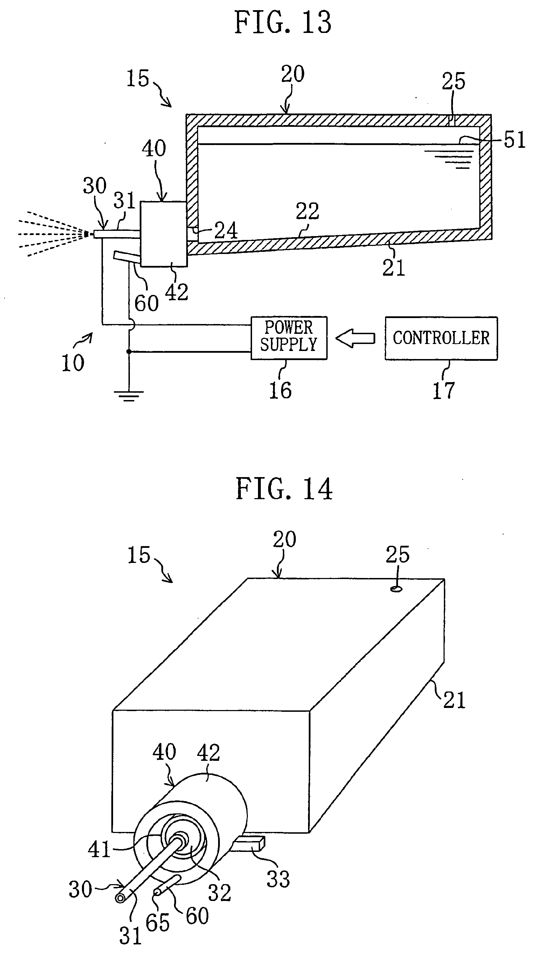

As shown in each of Figures **13** through **16**, the electrostatic spraying device **(10)** of the present embodiment employs, as a second electrode, a rod shaped electrode **(60)** as a substitute for the ring electrode **(35)** of the first embodiment. The rod shaped electrode **(60)** is a member shaped like a rod having a circular cross section and is formed of an electrically conducting material such as metal.

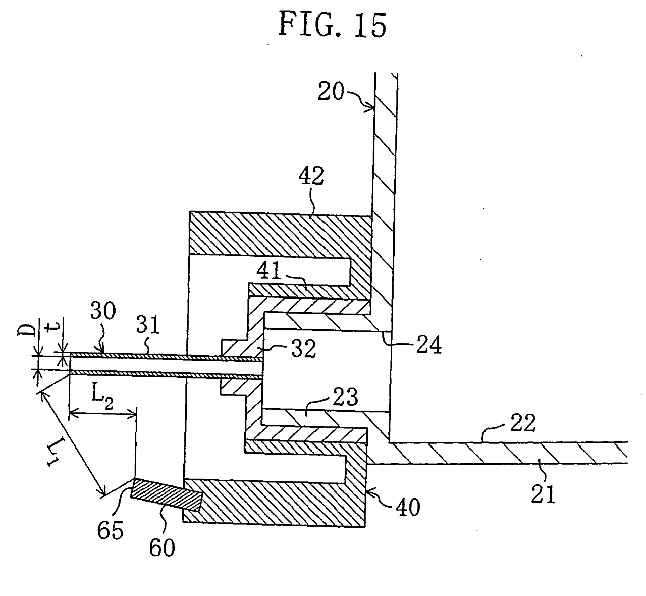

The rod shaped electrode **(60)** is mounted in a projecting manner on the tip end surface (left end surface in Figures **13** and **15****)** of the outer tubular part **(42)** of the electrode holder **(40).** The tip portion of the rod shaped electrode **(60)** projecting from the outer tubular part **(42)** constitutes the head end **(65).** The head end **(65)** of the rod shaped electrode **(60)** is situated nearer to the solution tank **(20)** than the tip of the spray nozzle **(31).** In other words, the rod shaped electrode **(60)** is arranged at a rearward position backed away from the tip of the spray nozzle **(31)**. In addition, the head end **(65)** of the rod shaped electrode **(60)** is positioned lateral to the spray nozzle **(31)**. And the rod shaped electrode **(60)** is electrically connected to the negative terminal of the power supply **(16)**.

In the present embodiment, the direct distance from the outer peripheral edge of the tip of the spray nozzle **(31)** to the outer peripheral edge of the head end **(65)** of the rod shaped electrode **(60),** i.e., the distance (L₁) between the tip of the spray nozzle **(31)** and the tip of the rod shaped electrode **(60)** (see Figure **15****)**, is preferably not less than 5.0 mm nor more than 20.0 mm. In addition, the horizontal distance from the tip of the spray nozzle **(31)** to the head end **(65)** of the rod shaped electrode **(60),** i.e., the backed-away distance (L₂) of the rod shaped electrode **(60)** from the tip of the spray nozzle **(31)** (see Figure **15**), is preferably not less than 3.0 mm nor more than 10.0 mm. The reason why these distances (L₁, L₂)should be set to fall within the aforesaid ranges is the same as mentioned in the first embodiment.

In the electrostatic spraying device **(10)** of the present embodiment, upon creation of an electric potential difference between the spray nozzle **(31)** and the rod shaped electrode **(60)** by turning on the power supply **(16),** an electric field is formed between the tip of the spray nozzle **(31)** and the head end **(65)** of the rod shaped electrode **(60)**. In other words, in the electrostatic spraying device **(10)** of the present embodiment, the electric field is formed between these relatively narrow regions. And in a space in the vicinity of the tip of the spray nozzle **(31)**, the electric field is concentrated in a relatively narrow region between the head end **(65)** of the rod shaped electrode **(60)** and the tip of the spray nozzle **(31)**.

There are cases where something having an electric potential lower than that of the spray nozzle **(31),** e.g., a human hand, may approach the spray nozzle **(31)** serving as a first electrode during operation of the electrostatic spraying device **(10).** In such a case, there is formed an electric field also between the human hand and the spray nozzle **(31)**, which electric filed disturbs the electric field formed in the vicinity of the tip of the spray nozzle **(31).** This may give rise to the possibility that spray from the spray nozzle **(31)** becomes unstable.

On the other hand, in the present embodiment, the rod shaped electrode **(60)** having the head end **(65)** is employed as a second electrode, whereby an electric field formed in the vicinity of the tip of the spray nozzle **(31)** is concentrated in a narrow region. As a result of such arrangement, even when somewhere in the vicinity of the tip of the spray nozzle **(31)** is subjected to a disturbance by, for example, a human hand approaching the spray nozzle **(31)**, the magnitude of such a disturbance is, as compared to the electric field formed in the vicinity of the tip of the spray nozzle **(31)**, small enough to enable the spray nozzle **(31)** to continue stably spray of the liquid.

In addition, in the present embodiment, the rod shaped electrode **(60)** is arranged at a position posterior to the tip of the spray nozzle **(31)**, thereby making it possible to prevent liquid sprayed from the tip of the spray nozzle **(31)** from adhering to the rod shaped electrode **(60)**. Consequently, it becomes possible to prevent the electric field between the rod shaped electrode **(60)** and the spray nozzle **(31)** from becoming unstabilized because of contamination of the rod shaped electrode **(60)** caused by liquid adhesion thereto. Therefore, in accordance with the present embodiment, the rod shaped electrode **(60)** is kept clean, thereby making it possible to stabilize the electric field between the rod shaped electrode **(60)** and the spray nozzle **(31)**, and spray of the liquid from the spray nozzle **(31)** is stably continued, as in the first embodiment.

### VARIATION OF THE SECOND EMBODIMENT

The electrostatic spraying device **(10)** of the present embodiment may have, as a second electrode, a triangular plate electrode **(61)** as a substitute for the rod shaped electrode **(60).**

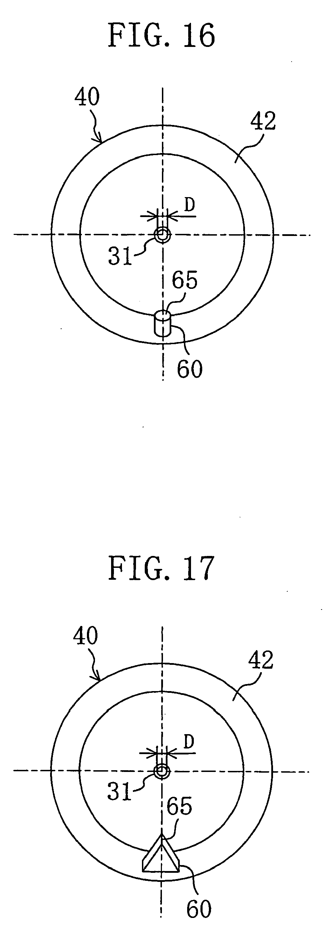

As shown in Figure 17, the triangular plate electrode **(61)** is shaped like a triangular plate. Like the rod shaped electrode **(60),** the triangular plate electrode **(61)** is mounted in a projecting manner on the tip end surface of the outer tubular part **(42)** of the electrode holder **(40).** A corresponding portion of the triangular plate electrode **(61)** to the bottom is embedded into the outer tubular part **(42)** while a corresponding portion thereof to the top is projected forwardly from the tip end surface of the outer tubular part **(42)**. And the portion of the triangular plate electrode **(61)** corresponding to the top constitutes the head end **(65).**

Upon creation of an electric potential difference between the spray nozzle **(31)** and the triangular plate electrode **(61)**, there is formed an electric field between the tip of the spray nozzle **(31)** and the head end **(65)** of the triangular plate electrode **(61).** And in a space in the vicinity of the tip of the spray nozzle **(31),** the electric filed is concentrated in a relatively narrow region between the head end **(65)** of the triangular plate electrode **(61)** and the tip of the spray nozzle **(31)**, whereby, even when the electric field is somewhat disturbed by external factors, spray from the tip of the spray nozzle **(31)** is stably continued.

As described above, in the present embodiment, it suffices if a member serving as a second electrode is provided with a projected portion (i.e., the head end **(65)**) and the member provided as a second electrode may have any entire shape.

### THIRD EMBODIMENT OF THE INVENTION

Description will be made in regard to a third embodiment of the present invention. The present embodiment is a modification of the first embodiment in that in the spray cartridge **(15)** the spray nozzle **(31)** is modified in shape. The spray nozzle **(31)** of the present embodiment is applicable to the spray cartridge **(15)** of the second embodiment.

The inside diameter of the spray nozzle **(31)** of the present embodiment is constant all over its full length. On the other hand, the spray nozzle **(31)** is formed in such a shape that in the vicinity of the tip of the spray nozzle **(31)** the outside diameter gradually becomes smaller towards the endmost portion, in other words the spray nozzle **(31)** is tapered in the vicinity of the tip thereof. In the spray nozzle **(31)**, the tapered portion constitutes the tip end **(31a).** And the tip end **(31a)** of the spray nozzle **(31)** has an endmost portion formed in an edge-like shape and its thickness at the endmost portion is substantially 0 mm.

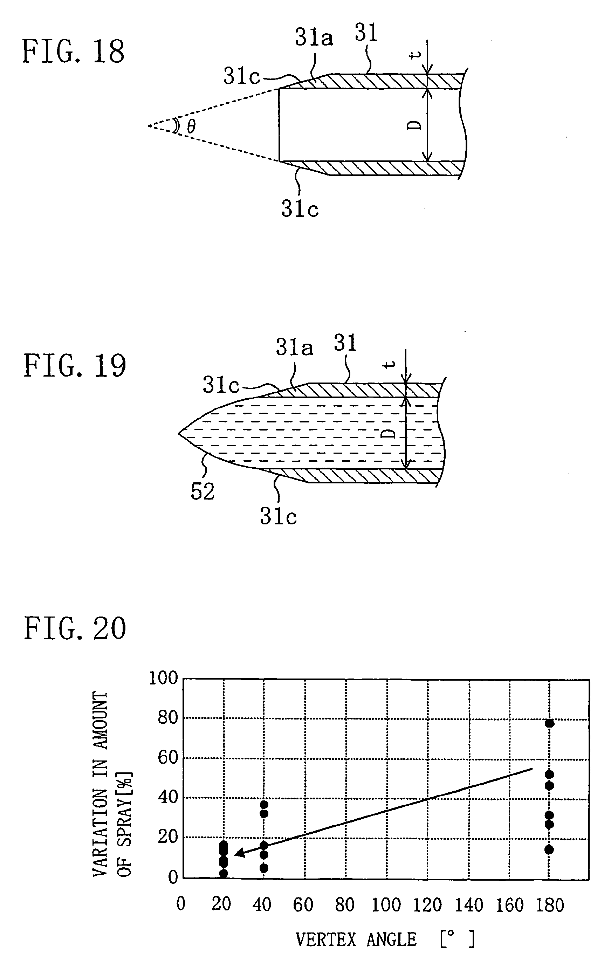

Description will be made in regard to the detailed shape of the spray nozzle **(31)** with reference to Figure **18**. The spray nozzle **(31)** has an outside diameter of 0.7 mm and an inside diameter (D) of 0.4 mm. The spray nozzle **(31)** has a thickness (t) of 0.15 mm. In addition, the vertex angle (angle θ shown in Figure **18****)** of the outer peripheral surface **(31c)** of the tip end **(31a)** formed in a tapered shape is 20 degrees. In other words, the outer peripheral surface **(31c)** of the tip end **(31a)** of the spray nozzle **(31)** is a cone shaped surface having a vertex angle of 20 degrees.

Upon application of a voltage to the spray nozzle **(31)**, an electric field is formed in the vicinity of the tip of the spray nozzle **(31)**, and liquid is sprayed from the spray nozzle **(31)**. At the same time, the gas-liquid interface **(52)** is extended to have a cone shape in the tip of the spray nozzle **(31),** as shown in Figure **19**.

The reason why the vertex angle (θ) of the tip end **(31a)** of the spray nozzle **(31)** is set at 20 degrees will be described with reference to Figure **20**.

Figure **20** is a graph showing variation in the volume of fluid sprayed from the spray nozzle **(31)** per unit time (variation in the amount of spray from the spray nozzle **(31))** when the vertex angle (θ) of the tip end **(31a)** is set at **20** degrees, when set at 40 degrees, and when set at 180 degrees, respectively. The spray nozzle **(31)** whose vertex angle is 180 degrees is one that has a constant thickness all over its full length and is not provided, in the vicinity of its tip, with a tapered surface (see Figures **4** and **6****).**

More specifically, measurement of variation in the amount of spray was carried out five times for each of three different types of spray nozzles **(31).** The measurement results show that the spray nozzle **(31)** whose tip end **(31a)** is tapered (vertex angle (θ): 20 or 40 degrees) underwent remarkably less variation in the amount of spray, in comparison with the non-tapered spray nozzle **(31)** (vertex angle (θ): 180 degrees). The measurement results further show that the spray nozzle **(31)** whose vertex angle (θ) is 20 degrees underwent less variation in the amount of spray, in comparison with the spray nozzle **(31)** whose vertex angle (θ) is 40 degrees. Therefore, in the present embodiment, the vertex angle (θ) of the tip end **(31a)** of the spray nozzle **(31)** is set at 20 degrees so as to minimize variation in the amount of spray.

### ADVANTAGEOUS EFFECTS OF THE THIRD EMBODIMENT

In the present embodiment, the thickness of the spray nozzle **(31)** at the endmost portion thereof is set at substantially 0 mm. This therefore makes it possible to keep the diameter of the gas-liquid interface **(52),** formed in the tip of the spray nozzle **(31)** during spray operation, approximately constant, whereby variation in the amount of spray in the electrostatic spraying device **(10)** is lessened. Therefore, the present embodiment eliminates flaws including, for example, unnecessary liquid consumption resulting from excess liquid atomization, insufficiency in the amount of spray, et cetera.

### FIRST VARIATION OF THE THIRD EMBODIMENT

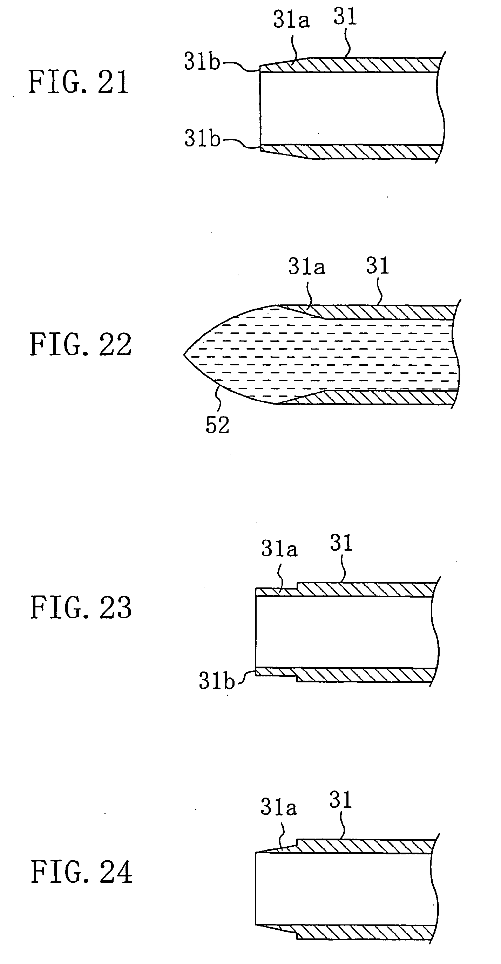

The endmost portion of the spray nozzle **(31)** of the present embodiment is formed in an edge-like shape, but it may be formed in a flat plate-like shape, as shown in Figure **21**. More specifically, although the outside diameter of the tip end **(31a)** of the spray nozzle **(31)** gradually becomes smaller towards the tip of the spray nozzle **(31)**, it is still greater, also in the endmost portion of the spray nozzle **(31),** than the inside diameter thereof. Stated another way, the thickness of the spray nozzle **(31)** in the endmost portion thereof is not 0 mm, and the ring-like tip end surface **(31b)** which is flat is formed.

### SECOND VARIATION OF THE THIRD EMBODIMENT

In the spray nozzle **(31)** of the present embodiment, the inner peripheral surface of the tip end **(31a)** may be a tapered surface spreading towards the endmost portion of the spay nozzle **(31),** as shown in Figure **22**. More specifically, in the endmost portion of the spray nozzle **(31)**, the outside diameter thereof becomes constant while on the other hand the inside diameter thereof gradually becomes greater towards the endmost portion of the spray nozzle **(31).** And the tip end **(31a)** of the spray nozzle **(31)** has an endmost portion which is formed in an edge-like shape and its thickness at the endmost portion is substantially 0 mm.

### THIRD VARIATION OF THE THIRD EMBODIMENT

In the spray nozzle **(31)** of the present embodiment, the tip end **(31a)** may be made thinner by one step, in comparison with the rest of the spray nozzle **(31)** other than the tip end **(31a),** as shown in Figure **23**. More specifically, in the spray nozzle **(31)**, the outside diameter of the tip end **(31a)** is smaller than that of the rest of the spray nozzle **(31)** other than the tip end **(31a)**. The outside diameter of the tip end **(31a)** is constant throughout its full length. In addition, the inside diameter of the tip end **(31a)** is constant throughout its full length. The difference between the inside and outside diameters of the tip end **(31a)** is smaller than the difference between the inside and outside diameters of the rest of the spray nozzle **(31)** other than the tip end **(31a).**

In the present variation, as shown in Figure **24****,** the outside diameter of the tip end **(31a)** may gradually become smaller towards the tip of the spray nozzle **(31)**.

### FOURTH EMBODIMENT OF THE PRESENT INVENTION

Description will be made in regard to a fourth embodiment of the present invention. The present embodiment is a modification of the first embodiment in that in the spray cartridge **(15)** the spray nozzle **(31)** is modified in configuration. The spray nozzle **(31)** of the present embodiment is applicable also to the spray cartridge **(15)** of the second embodiment.

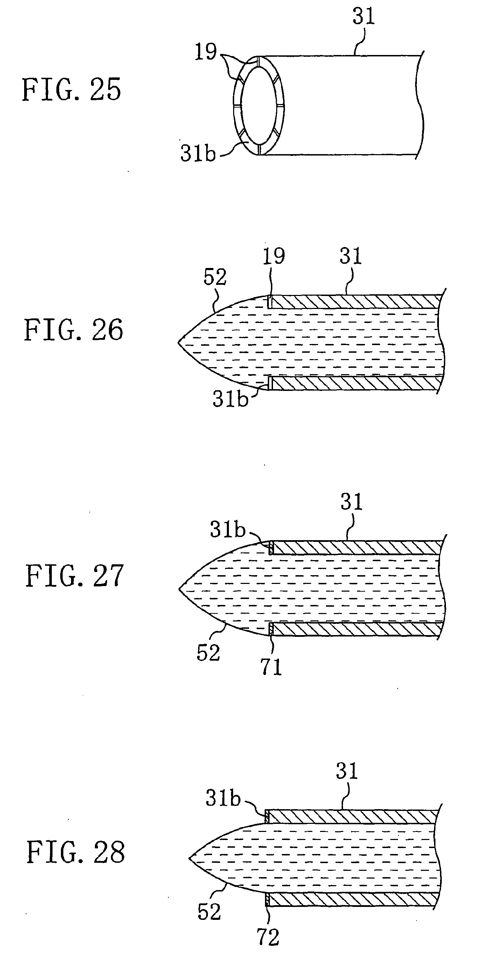

As shown in Figures **25** and **26****,** the tip end surface **(31b)** of the spray nozzle **(31)** of the present embodiment is subjected to a process for forming narrow grooves **(19)** as a hydrophilic treatment. More specifically, the tip end surface **(31b)** of the spray nozzle **(31)** is provided with eight grooves **(16)** which radially extend and which are equally spaced from each other. The width of each groove **(19)** is extremely small in comparison with the inner or outer peripheral length. Consequently, when the cone shaped gas-liquid interface **(52)** is formed in the tip of the spray nozzle **(31)** during spray operation, liquid is distributed throughout each groove **(19)** by capillary action and the entire tip end surface **(31b)** of the spray nozzle **(31)** is liable to enter the wet state.

In addition, in the first variation of the third embodiment (see Figure **21****)** or in the second variation of the third embodiment (see Figure **23**), the tip end surface **(31b)** thereof may be treated to become hydrophilic.

In the present embodiment, the tip end surface **(31b)** of the spray nozzle **(31)** is treated such that it becomes hydrophilic, so that the entire tip end surface **(31b)** is liable to enter the wet state during spray from the spray nozzle **(31).** And the cone shaped gas-liquid interface **(52)** formed in the tip of the spray nozzle **(31)** comes to have a shape in continuous connection with the outside surface of the spray nozzle **(31)**, as shown in Figure **26** and is held stable while keeping such a shape. Accordingly, the gas-liquid interface **(52)** formed in the tip of the spray nozzle **(31)** little varies in size, thereby making it possible to reduce variation in the amount of spray in the electrostatic spraying device **(10).** This therefore makes it possible to keep the gas-liquid interface **(52)**, formed in the tip of the spray nozzle **(31)** during spray operation, substantially constant in size, thereby lessening variation in the amount of spray in the electrostatic. spraying device **(10).**

### FIRST VARIATION OF THE FOURTH EMBODIMENT

In the present embodiment, the tip end surface **(31b)** of the spray nozzle **(31)** may be subjected to a surface roughening process as a hydrophilic treatment. The tip end surface **(31b)** of the spray nozzle **(31)** is subjected to a surface roughening process by which many fine convex and concave portions are formed in the tip end surface **(31b)**. As a result, the tip end surface **(31b)** is liable to enter the wet state.

### SECOND VARIATION OF THE FOURTH EMBODIMENT

In the present embodiment, as shown in Figure **27****,** a hydrophilic coating **(71)** may be formed on the tip end surface **(31b)** of the spray nozzle **(31)**. In other words, the tip end surface **(31b)** of the spray nozzle **(31)** may be subjected to a coating process, as a hydrophilic treatment, for forming the coating **(71)** of a hydrophilic material. As a hydrophilic material, titanic oxide (TiO₂) which is widely used as a photocatalyst may be employed.

### FIFTH EMBODIMENT OF THE INVENTION

Description will be made in regard to a fifth embodiment of the present invention. The present embodiment is a modification of the first embodiment in that in the spray cartridge **(15)** the spray nozzle **(31)** is modified in configuration. The spray nozzle **(31)** of the present embodiment is applicable also to the spray cartridge **(15)** of the second embodiment.

As shown in Figure **28**, a water-repellent coating **(72)** is formed on the tip end surface **(31b)**. In other words, the tip end surface **(31b)** of the spray nozzle **(31)** is subjected to a coating process, as a water-repellent process, for forming the coating (72) of a water-repellent material.

Since the water-repellent coating **(72)** formed on the tip end surface **(31b)** of the spray nozzle **(31)** repels water during spray operation, the entire tip end surface **(31b)** is held in the non-wet state (i.e., dry state). And the cone shaped gas-liquid interface **(52)** formed in the tip of the spray nozzle **(31)** comes to have a shape in continuous connection with the inside surface of the spray nozzle **(31),** as shown in Figure 28 and is held stable while keeping such a shape. Accordingly, the gas-liquid interface **(52)** formed in the tip of the spray nozzle **(31)** little varies in size, thereby making it possible to lessen variation in the amount of spray in the electrostatic spraying device **(10)**. This therefore makes it possible to keep the gas-liquid interface **(52)**, formed in the tip of the spray nozzle **(31)** during spray operation, substantially constant in size, thereby lessening variation in the amount of spray in the electrostatic spraying device **(10)**.

### ANOTHER EMBODIMENT OF THE INVENTION

The above-described embodiments may be configured as follows.

### FIRST VARIATION OF THE OTHER EMBODIMENT

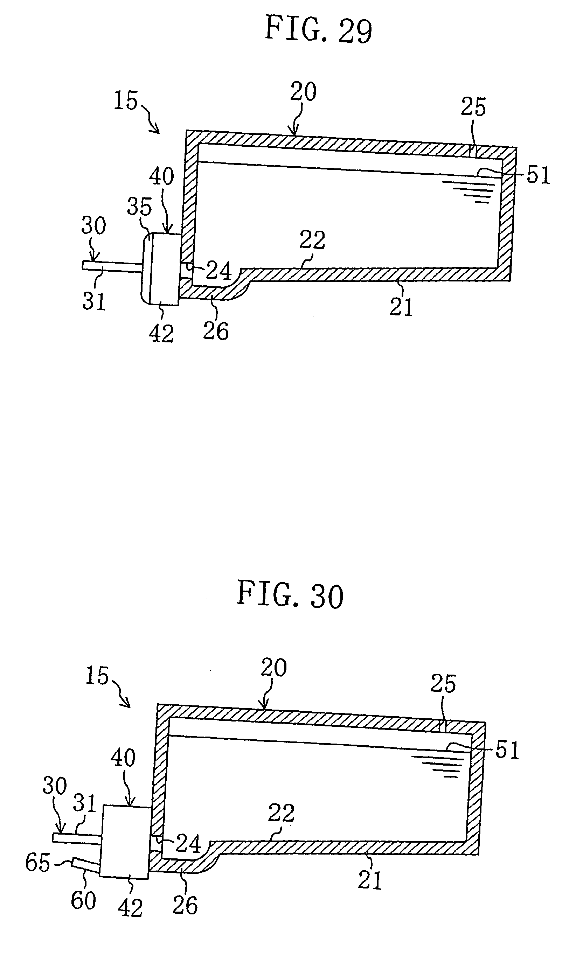

In the spray cartridge **(15)** of each of the above-described embodiments, the solution tank **(20)** may be constructed as follows. Referring now to Figures **29** and **30**, description will be made in regard to the solution tank **(20)** of the present variation. Figure **29** shows an application example in which the present variation is applied to the spray cartridge **(15)** of the first embodiment. Figure **30** shows another application example in which the present variation is applied to the spray cartridge **(15)** of the second embodiment.

The solution tank **(20)** of the present variation has a bulged part **(26).** The bulged part **(26)** is a portion which bulges out downwardly from the bottom of the tank body **(21)**. The bulged part **(26)** is situated adjacent to a side wall part on the side (lefthand side in Figures **29** and **30****)** of the front surface of the tank body **(21).** In addition, the bulged part **(26)** is formed by denting a portion of the bottom of the tank body **(21)** and its internal space is made lower by one step than the bottom surface **(22)** of the tank body **(21).**

In the solution tank **(20)** of the present variation, the position of the pipe part **(23)** is lower than the position of the pipe part **(23)** of each of the above-described embodiments. In addition, the through hole **(24)** for establishing fluid communication between the pipe part **(23)** and the inside of the tank body **(21)** is open at a position facing the internal space of the bulged part **(26).** The uppermost portion of the through hole **(24)** is lower than the bottom surface **(22)** of the tank body **(21)**. As in each of the above-described embodiments, also in the solution tank **(20)** of the present variation, the nozzle unit **(30)** is mounted to the pipe part **(23)**. And in the solution tank **(20)** of the present variation, the position of the tip of the spray nozzle **(31)** is lower than the bottom surface **(22)** of the tank body **(21)**.

In the solution tank **(20)** of the present variation, even when the tank body **(21)** contains therein only a very small amount of water solution (i.e., when water solution is left only in the internal space of the bulged part **(26)),** it is still possible for the tip of the spray nozzle **(31)** to be kept filled with water solution. Therefore, in accordance with the present variation, almost all of the water solution held within the tank body **(21)** can be sprayed, thereby suppressing the amount of water solution which remains unsprayed and thus wasted, to a minimum.

### SECOND VARIATION OF THE OTHER EMBODIMENT

The electrostatic spraying device **(10)** of each of the above-described embodiments may be configured such that the power supply **(16)** and the controller (1**7)** together constitute a body unit and the spray cartridge **(15)** is removable relative to the body unit. In the present variation, in the event that the solution tank **(20)** has become empty, the spray cartridge **(15)** which has been used up is detached from the body unit and a new spray cartridge **(15)** whose solution tank **(20)** is filled up with liquid is attached to the body unit.

In the present variation, it may be arranged such that not the entire spray cartridge **(15)** but a part thereof is removable relative to the body unit. For example, it may be arranged such that the solution tank **(20)** and the nozzle unit **(30)** are integrated and only the solution tank **(20)** and the nozzle unit **(30)** which are integral with each other are removable relative to the body unit. In other words, it may be arranged such that only the spray nozzle **(31)** which constitutes a first electrode and the solution tank **(20)** are removable relative to the body unit.

In the above case, the rest of the spray cartridge **(15)** other than the solution tank **(20)** and the nozzle unit **(30)** remain attached to the body unit. In other words, when the solution tank **(20)** becomes empty, it suffices merely to replace only the solution tank **(20)** and the nozzle unit **(30)**. Accordingly, the number of component parts that require replacement is reduced, thereby making it possible to cut down the cost for replacement, and the financial burden of a user is reduced. In addition, the spray nozzle **(31)** is also replaced together with the solution tank **(20)**, thereby making it possible to maintain the spray nozzle **(31)** in a clean state.

### THIRD VARIATION OF THE OTHER EMBODIMENT

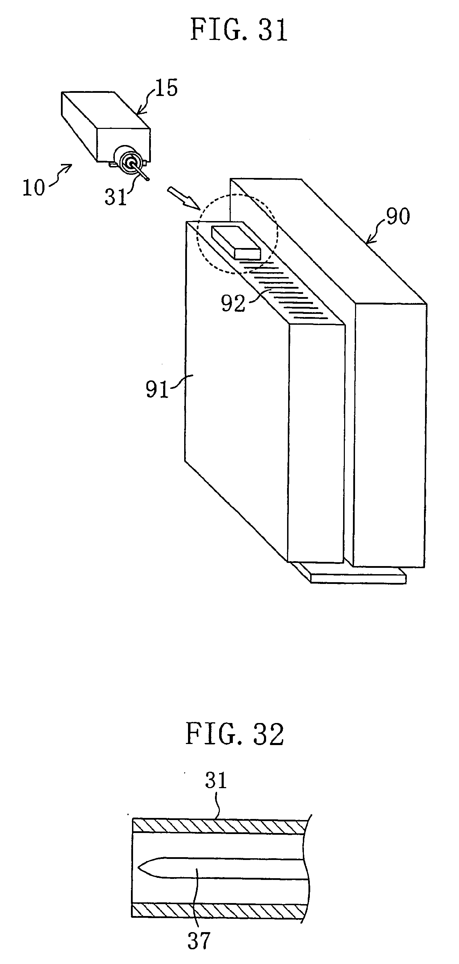

The electrostatic spraying device **(10)** of each of the above-described embodiments may be incorporated into an air cleaner **(90)** or into an air conditioner. Referring to Figure **31**, description will be made in regard to a case where the electrostatic spraying device **(10)** of any one of the above-described embodiments is mounted to the air cleaner **(90)**.

In the above case, the power supply and the controller for the electrostatic spraying device **(10)** are housed within a casing **(91)** of the air cleaner **(90)**. The spray cartridge **(15)** of the electrostatic spraying device **(10)** is removable relative to the casing **(91)** of the air cleaner **(90)**. When the spray cartridge **(15)** is mounted to the casing **(91)** of the air cleaner **(90)**, the tip of the spray nozzle **(31)** is situated above an air outlet **(92)** of the air cleaner **(90)**. And liquid droplets sprayed from the tip of the spray nozzle **(31)** are supplied, together with air blown out of the air cleaner **(90)**, into an indoor space. When the solution tank **(20)** becomes empty, the spray cartridge **(15)** is replaced with a new one.

In the present variation, it may be arranged such that not the entire spray cartridge **(15)** but a part thereof is removable relative to the air cleaner **(90)** or to the air conditioner. For example, it may be arranged such that the solution tank **(20)** and the nozzle unit **(30)** are integrated, and only the solution tank **(20)** and the nozzle unit **(30)** which are integral with each other are removable relative to the air cleaner **(90)**. In this case, as in the second variation, the cost of replacement is cut down, thereby making it possible to reduce the financial burden of a user. In addition, the spray nozzle **(31)** is also replaced together with the solution tank **(20),** thereby making it possible to maintain the spray nozzle **(31)** in a clean state.

### FOURTH VARIATION OF THE OTHER EMBODIMENT

In the spray cartridge **(15)** of each of the above-described embodiments, the electrode that is connected to the positive electrode of the power supply **(16)** may be provided separately from the spray nozzle **(31)**. For example, as shown in Figure **32**, the needle electrode **(37)** constituting a first electrode may be arranged in the inside of the spray nozzle **(31)** such that it lies coaxially with respect to the spray nozzle **(31)**. In this case, the needle electrode **(37)** and the ring electrode **(35)** are each formed of electrically conductive material while on the other hand the spray nozzle **(31)** is formed of non electrically conducting material. And upon application of a voltage to the needle electrode **(37)** in contact with water solution present in the spray nozzle **(31),** an electric field is formed in the vicinity of the tip of the spray nozzle **(31)**, and the water solution is atomized in the tip of the spray nozzle (31).

### FIFTH VARIATION OF THE OTHER EMBODIMENT

In the electrostatic spraying device **(10)** of each of the above-described embodiments, various liquids can be used as a liquid to be sprayed.

For example, a water solution of gamma-aminobutyric acid (GABA) which is a kind of amino acid can be used as a liquid to be sprayed. Gamma-aminobutyric acid is a kind of neurotransmitter and allegedly has tranquilization. In addition, a water solution of antioxidant agent such as catechin, proanthocyanidin, et cetera may be used. In addition, liquids containing a substance having a function of inhibiting the multiplication of microorganisms or of killing microorganisms may be used. In addition, liquids containing a substance capable of making odorous molecules present in the air odorless by chemical change such as neutralization may be used. Additionally, liquids containing a substance capable of chemically changing the antigenic site of protein which becomes an allergen may be used. In addition, liquids containing a substance capable of making harmful components present in the air harmless by chemical change may be used. Additionally, liquids containing various fragrant materials or substances capable of keeping harmful insects away may be used.

It should be noted that the above-descried embodiments are essentially preferable examples which are not intended in any sense to limit the scope of the present invention, its application, or its application range.

### INDUSTRIAL APPLICABILITY

As has been described above, the present invention finds its utility in the field of electrostatic spraying devices adapted and configured to atomize and spray liquid by electro hydrodynamic.

## Claims

1. An electrostatic spraying device comprising: a reservoir member (20) to store liquid; a tubular nozzle member (31) which is in fluid communication with an internal space of the reservoir member (20); a first electrode (31, 37) which is in contact with liquid present in the nozzle member (31); and a second electrode (35, 38, 60, 61) which is insulated from the liquid in the nozzle member (31),
wherein upon creation of an electric potential difference between the first electrode (31, 37) and the second electrode (35, 38, 60, 61) liquid is sprayed from the tip of the nozzle member (31);
wherein the tip of the nozzle member (31) is disposed at a lower position than a liquid level (51) in the reservoir member (20), and
wherein the nozzle member (31) includes a tip end surface (31b) which has been treated to become hydrophilic.

2. The electrostatic spraying device of claim 1, wherein the tip end surface (31b) of the nozzle member (31) has been subjected to a surface roughening process, as a hydrophilic treatment, for surface-roughening the tip end surface (31b).

3. The electrostatic spraying device of claim 1, wherein the tip end surface (31b) of the nozzle member (31) has been subjected to a process, as a hydrophilic treatment, for forming a groove (19) extending in the radial direction of the tip end surface (3 1 b).

4. The electrostatic spraying device of claim 1, wherein the tip end surface (31b) of the nozzle member (31) is subjected to a coating process, as a hydrophilic treatment, for forming a coating composed of a hydrophilic material on the tip end surface (3 1 b).

5. An electrostatic spraying device comprising: a reservoir member (20) to store liquid; a tubular nozzle member (31) which is in fluid communication with an internal space of the reservoir member (20); a first electrode (31, 37) which is in contact with liquid present in the nozzle member (31); and a second electrode (35, 38, 60, 61) which is insulated from the liquid in the nozzle member (31),
wherein upon creation of an electric potential difference between the first electrode (31, 37) and the second electrode (35, 38, 60, 61) liquid is sprayed from the tip of the nozzle member (31);
wherein the tip of the nozzle member (31) is disposed at a lower position than a liquid level (51) in the reservoir member (20), and
wherein the tip end surface (31b) of the nozzle member (31) has been subjected to a water-repellent treatment.

6. The electrostatic spraying device of claim 1 or 5, wherein the second electrode (35) is formed in a circular ring shape, said second electrode (35) being arranged coaxially with respect to the nozzle member (31).

7. The electrostatic spraying device of claim 1 or 5, wherein the second electrode (60, 61) is provided with a head end (65), said head end (65) being arranged to be positioned lateral to the nozzle member (31).

8. The electrostatic spraying device of claim 7 wherein the second electrode (60) is formed in a rod shape, said second electrode (60) having a tip which becomes the head end (65).

9. The electrostatic spraying device of claim 1 or 5, wherein the nozzle member (31) is composed of an electrically conductive material, said nozzle member (31) serving also as the first electrode.

10. The electrostatic spraying device of claim 9, wherein the distance between the tip of the nozzle member (31) and the second electrode (35, 38, 60, 61) is not less than 5 mm nor more than 20 mm.

11. The electrostatic spraying device of claim 9, wherein the tip of the nozzle member (31) has a thickness of not more than 0.2 mm.

12. The electrostatic spraying device of claim 9, wherein the nozzle member (31) has an inside diameter which is not less than 0.3 mm nor more than 1.0 mm.

13. The electrostatic spraying device of claim 9, wherein the second electrode (35, 38, 60, 61) is disposed at a rearward position backed away from the tip towards the base end of the nozzle member (31).

14. The electrostatic spraying device of claim 13, wherein the distance for which the second electrode (35, 38, 60, 61) is backed away from the tip of the nozzle member (31) is not less than 3 mm nor more than 10 mm.

15. The electrostatic spraying device of claim 1 or 5, wherein the nozzle member (31) includes a tip end (31 a) which has a smaller thickness than the rest of the nozzle member (31).

16. The electrostatic spraying device of claim 1 or 5, wherein the thickness of the tip end (31 a) of the nozzle member (31) gradually becomes reduced towards the tip of the nozzle member (31).

17. The electrostatic spraying device of claim 16, wherein the inside diameter of the tip end (31 a) of the nozzle member (31) is constant while on the other hand the outside diameter thereof gradually becomes reduced towards the tip of the nozzle member (31).
